# EUROPEAN PATENT APPLICATION

(11) **EP 4 446 423 A1**
(43) Date of publication of application: **16.10.2024**
(21) Application number: 22904207.2
(22) Date of filing: 06.12.2022
(51) Int. Cl.: C12P 21/02, A01H 1/00, A01H 5/00, A01H 6/82, C07K 14/78, C07K 19/00, C12N 9/90

(54) **METHOD FOR PRODUCING PROTEIN**

(30) Priority: 06.12.2021 JP 2021197463
(71) Applicant: AJINOMOTO CO., INC., Chuo-ku Tokyo 104-8315 (JP)
(72) Inventor: HIRANO, Hiroto, Kawasaki-shi, Kanagawa 210-8681 (JP); OZAKI, Sato, Kawasaki-shi, Kanagawa 210-8681 (JP)
(74) Representative: Hoffmann Eitle
(86) International application number: PCT/JP2022/044832
(87) International publication number: WO 2023/106279

(57) **Abstract**

A method for producing a target protein using a plant as an expression host is provided. The target protein is produced by expressing the target protein using a plant modified to express ERp27 and/or TMX1 as an expression host.

## Description

### Technical Field

The present invention relates to a method for producing a protein by using a plant as an expression host.

### Background Art

Plants are used as hosts for expression of recombinant proteins.

For mammals, such as humans, various proteins belonging to the protein disulfide isomerase (PDI) family, such as ERp27 and TMX1, are known (Non-patent document 1). Functions of ERp27 are unknown. TMX1 may have activities for catalyzing reactions that form, cleave, and rearrange disulfide bonds of proteins.

### Prior Art Reference

### Non-patent document

Non-patent document 1: James J Galligan, Dennis R Petersen. The human protein disulfide isomerase gene family. Hum Genomics. 2012 Jul 5;6(1):6

### Summary of the Invention

### Object to be achieved by the invention

An object of the present invention is to provide a novel technique for improving production of proteins using plants as expression hosts.

### Means for achieving the object

The inventors of the present invention found that modifying plants to express ERp27 or TMX1, which is classified into the protein disulfide isomerase (PDI) family, markedly increases expression amounts of target proteins in plants, and thus accomplished the present invention.

The present invention can be embodied, for example, as follows
[1] A method for producing a target protein, wherein
   the method comprises a step of expressing a gene encoding the target protein in a plant having the gene,
   wherein the plant has been modified so as to express ERp27 and/or TMX1, with the proviso that the target protein is expressed in a form containing a signal peptide at the N-terminus when the plant has been modified to express only TMX1 among ERp27 and TMX1.
[2] The method described above, wherein the target protein is expressed in a form containing a signal peptide at the N-terminus.
[3] The method described above, wherein the signal peptide has such a function that the target protein expressed in a form having the signal peptide at the N-terminus in the plant is translocated or localized to the endoplasmic reticulum.
[4] The method described above, wherein the ERp27 is a protein described in the following (a), (b), or (c):
   (a) a protein comprising the amino acid sequence shown as SEQ ID NO: 21;
   (b) a protein comprising an amino acid sequence of SEQ ID NO: 21 but which includes substitution, deletion, insertion, and/or addition of 1 to 10 amino acid residues, and having a function of increasing production of the target protein by the plant when expressed in the plant; or
   (c) a protein comprising an amino acid sequence having at least 90% identity to the amino acid sequence shown as SEQ ID NO: 21, and having a function of increasing production of the target protein by the plant when expressed in the plant.
[5] The method described above, wherein the TMX1 is a protein described in the following (a), (b), or (c):
   (a) a protein comprising the amino acid sequence shown as SEQ ID NO: 23;
   (b) a protein comprising an amino acid sequence of SEQ ID NO: 23 but which includes substitution, deletion, insertion, and/or addition of 1 to 10 amino acid residues, and having a function of increasing production of the target protein by the plant when expressed in the plant; or
   (c) a protein comprising an amino acid sequence having at least 90% identity to the amino acid sequence shown as SEQ ID NO: 23, and having a function of increasing production of the target protein by the plant when expressed in the plant.
[6] The method described above, wherein the plant is a plant of the family *Solanaceae.*
[7] The method described above, wherein the plant is benthamiana tobacco (*Nicotiana benthamiana*) or tobacco (*Nicotiana tabacum*)*.*
[8] The method described above, which further comprises a step of collecting the target protein.
[9] The method described above, wherein the step of collecting comprises a step of extracting the target protein from the plant.
[10] The method described above, wherein the target protein is extracted from a leaf of the plant.
[11] The method described above, wherein
   the extracting is performed in the presence of an active ingredient, and
   the active ingredient is an antioxidant and/or a metal sequestering agent.
[12] The method described above, wherein the active ingredient is L-ascorbic acid.
[13] The method described above, wherein the target protein is expressed and extracted in a form containing an HA tag.
[14] The method described above, wherein the HA tag is a peptide described in the following (a), (b), or (c):
   (a) a peptide comprising the amino acid sequence shown as SEQ ID NO: 17, 18, or 19;
   (b) a peptide comprising an amino acid sequence of SEQ ID NO: 17, 18, or 19 but which includes substitution, deletion, insertion, and/or addition of 1 to 5 amino acid residues, containing a tyrosine residue, and having a function of increasing production of the target protein by the plant when added to the target protein; or
   (c) a peptide comprising an amino acid sequence having at least 50% identity to the amino acid sequence shown as SEQ ID NO: 17, 18, or 19, containing a tyrosine residue, and having a function of increasing production of the target protein by the plant when added to the target protein.
[15] The method described above, wherein the HA tag contains 1 to 5 tyrosine residues.
[16] The method described above, wherein the target protein is a heterologous protein.
[17] The method described above, wherein the target protein is a human-derived protein.
[18] The method described above, wherein the target protein is a multimeric protein.
[19] The method described above, wherein the target protein is one or more types of proteins selected from an extracellular protein, an antibody-related molecule, a Notch ligand, and GFP.
[20] The method described above, wherein the target protein is laminin or a partial sequence thereof.
[21] The method described above, wherein the target protein is laminin 511E8.
[22] The method described above, wherein the target protein is an Fc fusion protein.
[23] The method described above, wherein the target protein is DLL4-Fc.
[24] The method described above, wherein the step of collecting further comprises a step of collecting the target protein from an extract obtained by the extracting.
[25] The method described above, wherein the plant has been further modified to express a chaperone.
[26] The method described above, wherein the chaperone is O-fucosyltransferase 1.
[27] The method described above, wherein the chaperone is a protein described in the following (a), (b), or (c):
   (a) a protein comprising the amino acid sequence shown as SEQ ID NO: 25;
   (b) a protein comprising an amino acid sequence of SEQ ID NO: 25 but which includes substitution, deletion, insertion, and/or addition of 1 to 10 amino acid residues, and having an O-fucosyltransferase 1 activity; or
   (c) a protein comprising an amino acid sequence having at least 90% identity to the amino acid sequence shown as SEQ ID NO: 25 and having an O-fucosyltransferase 1 activity.

### Brief Descriptions of the Drawings

[Fig. 1] Diagrams (photographs) showing the results of co-expression of DLL4-Fc and proteins of the protein disulfide isomerase (PDI) family. "M" indicates molecular weight markers. "Std" indicates a DLL4-Fc standard sample.
[Fig. 2] Diagram (photograph) showing the results of co-expression of human laminin 511E8 (denoted as "LN" in the diagram) and proteins of the protein disulfide isomerase (PDI) family. "M" indicates molecular weight markers. "Std" indicates a human laminin 511E8 standard sample.
[Fig. 3] Diagram (photograph) showing the results of co-expression of GFP and proteins of the protein disulfide isomerase (PDI) family. "M" indicates molecular weight markers.
[Fig. 4] Diagrams (photographs) showing the results of co-expression of DLL4-Fc and POFUT1 and co-expression of DLL4-Fc, and ERp27 and POFUT1. "M" indicates molecular weight markers. "Std" indicates a DLL4-Fc standard sample.

### Modes for Carrying out the Invention

The method of the present invention is a method for producing a target protein by using a plant as an expression host.

The method of the present invention may be specifically a method for producing a target protein, wherein the method comprises a step of expressing a gene encoding the target protein in a plant having the gene, and the plant has been modified so as to express ERp27 and/or TMX1.

The plant mentioned above (i.e., plant used in the method of the present invention) is also referred to as "plant of the present invention. The plant of the present invention is also referred to as "host" or "expression host".

### <1> Plant of the present invention

The plant of the present invention has a gene encoding a target protein. The gene encoding a target protein is also referred to as "target protein gene".

The plant of the present invention has an ability to produce a target protein. The plant of the present invention has an ability to produce a target protein, at least because it has a gene encoding the target protein. The plant of the present invention may have an ability to produce a target protein solely because it has a gene encoding the target protein, or may have an ability to produce a target protein because of a combination of having a gene encoding the target protein and another property (e.g., having been modified to express ERp27 and/or TMX1). The term "ability to produce a target protein" refers to an ability to express a target protein gene and thereby produce the target protein. The "ability to produce a target protein" may specifically mean an ability to express a target protein gene and thereby produce the target protein when the plant is cultured under appropriate conditions. The term "expression of a target protein gene" may be used interchangeably with "expression of a target protein". The expressed target protein may accumulate within or outside the plant. The expressed target protein may accumulate, especially, within the plant. That is, the "ability to produce a target protein" may mean, for example, an ability to express a target protein and accumulate it in the plant. The "ability to produce a target protein" may specifically mean, for example, an ability to express a target protein and accumulate it within the plant when the plant is cultured under appropriate conditions. Examples of accumulation within the plant include accumulation in cells of the plant or in extracellular apoplast of the plant. Examples of accumulation outside the plant include accumulation in the culture medium. The plant of the present invention may produce a greater amount of the target protein than the unmodified plant. The term "unmodified plant" means a plant that has not been modified to express ERp27 and/or TMX1. The amount of the target protein produced by the plant of the present invention is not particularly limited. The plant of the present invention may produce the target protein to such an extent that, for example, the target protein can be collected. The amount of the target protein produced by the plant of the present invention (e.g., accumulation amount of the protein in the plant) may be, for example, 0.0001 mg or more, 0.001 mg or more, 0.01 mg or more, 0.1 mg or more, or 1 mg or more, and 100 mg or less, 50 mg or less, or 10 mg or less per 1 g dry weight of the plant, or in any range defined by a combination of these minimum and maximum amounts.

The plant is not particularly limited as long as it can produce a target protein.

Examples of the plant include plants of the families *Solanaceae, Asteraceae, Cucurbitaceae, Orchidaceae, Poaceae,* and *Brassicaceae.* Examples of the plants of the family *Solanaceae* include plants of the genera *Nicotiana, Solanum,* and *Capsicum.* Examples of the plants of the genus *Nicotiana* include Benthamiana tobacco (*Nicotiana benthamiana*) and tobacco (*Nicotiana tabacum*)*.* Examples of the plants of the genus *Solanum* include tomato (*Solanum lycopersicum*)*,* eggplant (*Solanum melongena*)*,* and potato (*Solanum tuberosum*)*.* Examples of the plants of the genus *Capsicum* include *Capsicum annuum.* Examples of the plants of the family *Asteraceae* include plants of the genus *Lactuca.* Examples of the plants of the genus *Lactuca* include Lettuce (*Lactuca sativa*)*.* Examples of the plants of the family *Cucurbitaceae* include plants of the genus *Cucumis.* Examples of the plants of the genus *Cucumis* include melon (*Cucumis melo*)*.* Examples of the plants of the family *Orchidaceae* include plants of the genus *Phalaenopsis.* Examples of the plants of the genus *Phalaenopsis* include orchid (*Phalaenopsis Aphrodite*)*.* Examples of the plants of the family *Poaceae* include plants of the genera *Oryza* and *Zea.* Examples of the plants of the genus *Oryza* include rice (*Oryza sativa*)*.* Examples of the plants of the genus *Zea* include maize (*Zea mays*)*.* Examples of the plants of the family *Brassicaceae* include plants of the genus *Arabidopsis.* Examples of the plants of the genus *Arabidopsis* include *Arabidopsis thaliana.* Examples of the plants include, especially, plants of the genus *Nicotiana,* such as benthamiana tobacco and tobacco.

The term "plant" may mean the whole plant body or a part of plant body. That is, for both expression and extraction of a target protein, the whole plant body or a part of plant body may be used. Examples of the part of plant body include plant cells, callus, seed, bud, leaf, stem, trunk, root, petal, and fruit. Examples of the part of the plant body include, especially, leaf.

The target protein is not particularly limited as long as it can be expressed by a plant as a host. The protein may be a protein derived from the host or a heterologous protein. The term "heterologous protein" means a protein that is exogenous to the host that expresses the protein (i.e., the plant that expresses the target protein). The "heterologous protein" may specifically mean a protein that is expressed from a gene that is not native to the host and introduced into the host. The target protein may be, for example, a naturally occurring protein, a protein modified from it, or a protein having an artificially designed amino acid sequence. The target protein may be, for example, a protein of microbial origin, a protein of plant origin, a protein of animal origin, or a protein of viral origin. The target protein may be, especially, a protein of human origin. The target protein may be a monomeric protein or a multimeric protein. The multimeric protein may be a homomultimer consisting of a single type of subunits or a heteromultimer consisting of two or more types of subunits. The target protein may be a secretory protein or a non-secretory protein. The term "protein" also encompasses those called peptides, such as oligopeptides and polypeptides.

As the target protein, one type of protein may be produced, or two or more types of proteins may be produced. If the target protein is a heteromultimer, one type of subunit among the subunits constituting the heteromultimer may be produced, or two or more types, e.g., all types, of the subunits may be produced.

Specific examples of the target protein include enzymes, physiologically active proteins, receptor proteins, antigenic proteins, and any other proteins.

Examples of the enzymes include cellulase, xylanase, transglutaminase, protein-glutaminase, protein-asparaginase, isomaltodextranase, protease, endopeptidase, exopeptidase, aminopeptidase, carboxypeptidase, collagenase, chitinase, γ-glutamylvaline synthase, glutamate-cysteine ligase, and glutathione synthetase.

Examples of the physiologically active proteins include growth factors, hormones, cytokines, antibody-related molecules, and antibody mimetics.

Examples of the growth factors include epidermal growth factor (EGF), insulin-like growth factor-1 (IGF-1), transforming growth factor (TGF), nerve growth factor (NGF), brain-derived neurotrophic factor (BDNF), vascular endothelial growth factor (VEGF), granulocyte-colony stimulating factor (G-CSF), granulocyte-macrophage-colony stimulating factor (GM-CSF), platelet-derived growth factor (PDGF), erythropoietin (EPO), thrombopoietin (TPO), acidic fibroblast growth factor (aFGF or FGF1), basic fibroblast growth factor (bFGF or FGF2), fibroblast growth factor (FGF-4), keratinocyte growth factor (KGF-1 or FGF7, or KGF-2 or FGF10), hepatocyte growth factor (HGF), stem cell factor (SCF ), and activin. Examples of activin include activins A, C, and E.

Examples of the hormones include insulin, glucagon, somatostatin, human growth hormone (hGH), parathyroid hormone (PTH), calcitonin, and exenatide.

Examples of the cytokines include interleukins, interferons, and tumor necrosis factor (TNF).

The physiologically active proteins may be an entire protein or a part thereof. Examples of the part of a protein include, for example, a physiologically active part. Specific examples of the physiologically active part include, for example, the physiologically active peptide, teriparatide, which consists of the N-terminus 34 amino acid residues of the mature parathyroid hormone (PTH).

The term "antibody-related molecule" may refer to a protein that contains a molecular species consisting of a single domain or a combination of two or more domains selected from domains constituting a complete antibody. Examples of the domains constituting a complete antibody include the domains of the heavy chain, VH, CH1, CH2, and CH3, and the domains of the light chain, VL and CL. The antibody-related molecules may be a monomeric or multimeric protein as long as they contain the molecular species described above. If the antibody-related molecules are a multimeric protein, they may be a homomultimer consisting of a single type of subunits or a heteromultimer consisting of two or more types of subunits. Specific examples of the antibody-related molecules include complete antibodies, Fab, F(ab'), F(ab')₂ , Fc, dimer consisting of heavy chain (H chain) and light chain (L chain), Fc fusion protein, heavy chain (H chain), light chain (L chain), single chain Fv (scFv), sc(Fv)₂, disulfide-linked Fv (sdFv), diabody, and VHH fragment (Nanobody (registered trademark)). More specific examples of the antibody-related molecules include trastuzumab, adalimumab, nivolumab, VHH antibody N15, and VHH antibody 9g8. Examples of the Fc fusion protein include fusion proteins of various target proteins exemplified in this description and the Fc region. Specific examples of the Fc fusion protein include fusion proteins of Notch ligand and Fc region described later. Examples of the fusion proteins of Notch ligand and Fc region include DLL4-Fc (namely, the fusion protein of DLL4 and Fc region).

The term "antibody mimetic" may refer to an organic compound that can specifically bind to an antigen but is not structurally related to an antibody. Specific examples of the antibody mimetics include the Z domain of protein A (Affibody). More specific examples of the antibody mimetics include the ZHER2 Affibody.

Examples of the receptor proteins include receptors for physiologically active proteins and other physiologically active substances. Examples of such other physiologically active substances include neurotransmitters such as dopamine. The receptor proteins may be an orphan receptor for which corresponding ligand is not known.

The antigenic proteins are not particularly restricted so long as they can elicit an immune response. The antigenic protein can be appropriately selected according to, for example, the target of the intended immune response. The antigenic proteins can be used, for example, as vaccines.

Examples of the other proteins include liver-type fatty acid-binding protein (LFABP), fluorescent proteins, immunoglobulin-binding proteins, albumins, Notch ligands, fibroin-like proteins, and extracellular proteins. Examples of the fluorescent proteins include green fluorescent protein (GFP) and monomeric red fluorescent protein (mRFP). Examples of the immunoglobulin-binding proteins include protein A, protein G, and protein L. Examples of the albumins include human serum albumin. Examples of the Notch ligands include DLL1, DLL3, DLL4, Jagged-1, and Jagged-2. The Notch ligands may be constituted as, for example, a fusion protein with the Fc region. Examples of the fibroin-like proteins include those disclosed in WO2017/090665 and WO2017/171001.

Examples of the extracellular proteins include fibronectin, vitronectin, collagen, osteopontin, laminin, and partial sequences thereof. Laminin is a protein having a heterotrimeric structure consisting of the α-chain, β-chain, and γ-chain. Examples of laminin include mammalian laminin. Examples of the mammals include primates such as human, monkey, and chimpanzee; rodents such as mouse, rat, hamster, and guinea pig; and other various mammals such as rabbit, horse, cattle, sheep, goat, pig, dog, and cat. Examples of the mammals include, especially, human. Examples of the subunit chains of laminin (i.e., α-chain, β-chain, and γ-chain) include 5 types of α-chains (α1 to α5), three types of β-chains (β1 to β3), and three types of γ-chains (γ1 to y3). Combinations of these subunit chains constitute various isoforms of laminin. Specifically, examples of laminin include, for example, laminin 111, laminin 121, laminin 211, laminin 213, laminin 221, laminin 311, laminin 321, laminin 332, laminin 411, laminin 421, laminin 423, laminin 511, laminin 521, and laminin 523. The numbers in the names of laminin indicate, from left to right, the types of α-chain, β-chain, and γ-chain. That is, for example, "laminin 511" means a protein having a heterotrimeric structure consisting of the α5-chain, β1-chain, and γ1-chain. Examples of the partial sequences of laminin include laminin E8, which is the E8 fragment of laminin. The laminin E8 is specifically a protein having a heterotrimeric structure consisting of the E8 fragment of the α-chain (α-chain E8), E8 fragment of the β-chain (β-chain E8), and E8 fragment of the γ-chain (γ-chain E8). The subunit chains of laminin E8 (i.e., α-chain E8, β-chain E8, and γ-chain E8) are also collectively referred to as "E8 subunit chains". Examples of the E8 subunit chains include the E8 fragments of the laminin subunit chains exemplified above. Laminin E8 presents as various isoforms consisting of combinations of these E8 subunit chains. Specific examples of laminin E8 include laminin 111E8, laminin 121E8, laminin 211E8, laminin 221E8, laminin 332E8, laminin 421E8, laminin 411E8, laminin 511E8, and laminin 521E8. The numbers in the names of laminin E8 indicate, from left to right, the types of α-chain, β-chain, and γ-chain. That is, for example, "laminin 511E8" means the E8 fragments of laminin 511, specifically, a protein having a heterotrimeric structure consisting of the E8 fragment of the α5-chain (α5-chain E8), E8 fragment of the β1-chain (β1-chain E8), and E8 fragment of the γ1-chain (γ1 chain E8).

The term "α-chain E8" may mean a region near the C-terminus of the α-chain, specifically, the C-terminus fragment of the α-chain excluding the globular domains 4 and 5, more specifically, a fragment consisting of the C-terminus 780 to 830 (e.g., 790 to 800) amino acid residues of the α-chain excluding the globular domains 4 and 5. The term "β-chain E8" may mean a C-terminus fragment of the β-chain, more specifically, a fragment consisting of C-terminus 220 to 230 amino acid residues of the β-chain. The term "γ-chain E8" may mean a C-terminus fragment of the γ-chain, specifically, a fragment consisting of C-terminus 240 to 250 amino acid residues of the γ-chain. Examples of the human α5-chain E8 include the site of the amino acid numbers 2534 to 3327 of GenBank Accession No. NP_005551. Examples of the human β1-chain E8 include the site of the amino acid numbers 1561 to 1786 of GenBank Accession No. NP_002282. Examples of the human γ1-chain E8 include the site of the amino acid numbers 1364 to 1609 of GenBank Accession No. NP_002284.

The target protein gene may be, for example, a gene having a known or natural nucleotide sequence of a gene encoding any of the proteins described above. Similarly, the target protein may be, for example, a protein having a known or natural amino acid sequence of any of the proteins described above. Examples of known or natural nucleotide and amino acid sequences include those registered in public databases such as NCBI and those described in various patent documents and non-patent references. The target protein gene may also be, for example, a variant of a gene having a known or natural nucleotide sequence of a gene encoding any of the proteins described above. Similarly, the target protein may be, for example, a variant of a protein having a known or natural amino acid sequence of any of the proteins described above. The variant is not particularly limited as long as it has desired functions. The variant may be, for example, a conservative variant. For variants (e.g., conservative variants) of the target protein and target protein gene, the descriptions for variants (e.g., conservative variants) of ERp27, TMX1 and the genes encoding them mentioned below can be applied. For example, the target protein gene may be a gene encoding a protein having an amino acid sequence derived from a known or natural amino acid sequence of any of the proteins described above by substitution, deletion, insertion or addition of one or several amino acids at one or several positions. The target protein gene may also be, for example, a gene encoding a protein having an amino acid sequence that has at least 80% identity, preferably at least 90% identity, more preferably at least 95% identity, further preferably at least 97% identity, especially preferably at least 99% identity, to the entire sequence of a known or natural amino acid sequence of any of the proteins described above. Proteins identified with a species of origin are not limited to proteins found in that species itself, but also include proteins having the amino acid sequences of the proteins found in the species and variants thereof. The variants may or may not be found in the species. That is, for example, "human-derived proteins" are not limited to proteins found in humans per se, but shall include proteins having the amino acid sequences of the proteins found in humans and variants thereof. The gene encoding a target protein may include replacement of a codon with an equivalent codon. For example, the gene encoding a target protein may be modified to have codons optimized according to frequencies of codons found in the host to be used.

The target protein may contain another amino acid sequence in addition to the amino acid sequence of such a target protein as exemplified above. Such another amino acid sequence is also referred to as "additional sequence". That is, the target protein may be a fusion protein with an additional sequence. Unless otherwise noted, the expression that "the target protein contains an additional sequence" or "the target protein is a fusion protein with an additional sequence" means that the target protein contains an additional sequence at the time of expression, extraction, and/or finally (i.e., the target protein expressed, extracted, and/or finally obtained contains an additional sequence). The term "target protein extracted" or "target protein at the time of extraction" may be synonymous with the term target protein accumulated in the plant (i.e., contained within the plant). In other words, the target protein gene may contain a nucleotide sequence encoding an additional sequence in addition to the nucleotide sequence of such a target protein gene as exemplified above.

The target protein may be expressed, for example, in a form containing an additional sequence (i.e., as a fusion protein with an additional sequence) and lose a part or all of the additional sequence at the time of extraction or in the final product. That is, the target protein may contain an additional sequence at the time of expression and may not contain a part or all of the additional sequence at the time of extraction or in the final product. The expression "the target protein is expressed in a form containing an additional sequence" or "the target protein contains an additional sequence at the time of expression" means that the target protein contains the additional sequence at least at the time of expression, and does not necessarily mean that the target protein at the time of extraction or the target protein finally obtained contains the additional sequence.

The target protein may be, for example, expressed and extracted in a form containing an additional sequence (i.e., as a fusion protein with an additional sequence) and finally lose a part or all of the additional sequence. That is, the target protein may contain the additional sequence at the time of expression and extraction, and may not finally contain a part or all of the additional sequence. The expression "the target protein is expressed and extracted in a form containing an additional sequence" or "the target protein contains an additional sequence at the time of expression and extraction" means that the target protein contains the additional sequence at least at the time of expression and extraction, and does not necessarily mean that the target protein finally obtained contains the additional sequence.

The other amino acid sequence is not particularly limited as long as it does not impair the purpose of the present invention. The additional sequence can be selected according to various conditions, such as, for example, the intended use thereof. Examples of the additional sequence include signal peptide (also called signal sequence), peptide tag, and protease recognition sequence. The additional sequence may be linked to, for example, the N-terminus, C-terminus, or both of the target protein. When an additional sequence is linked to the N-terminus of the target protein, the amino acid sequence of the target protein before the linkage of the additional sequence may be one in which the N-terminal amino acid residue has been removed. Examples of the N-terminal amino acid residue include initial methionine residue. As the additional sequence, one type of amino acid sequence may be used, or a combination of two or more types of amino acid sequences may be used.

The signal peptide is not particularly limited so long as it functions in the plant that expresses the target protein. Examples of the signal peptide include the signal peptide of alkaline chitinase (basic chitinase), signal peptide of granule-bound starch synthase (GBSS), signal peptide of the small subunit of ribulose-1,5-bisphosphate carboxylase/oxygenase (RuBisCo), and signal peptide of butyrylcholinesterase (BChE). Examples of the signal peptide also include signal peptides of various organisms including plants. Examples of the plants include *Arabidopsis thaliana.* Amino acid sequences of signal peptides of various organisms and nucleotide sequences encoding them can be obtained from, for example, public databases such as NCBI and technical literature such as patent documents. The amino acid sequence of the signal peptide of alkaline chitinase of *Arabidopsis thaliana* is shown as SEQ ID NO: 1. The signal peptide may be utilized, for example, to translocate or localize the expressed target protein to a specific intracellular organelle or intracellular compartment. Specifically, the signal peptide may be utilized, for example, to translocate or localize the expressed target protein to the endoplasmic reticulum. In other words, any of the signal peptides exemplified above may function as a signal for translocation to the endoplasmic reticulum. The target protein may contain a signal peptide at the time of expression. That is, the target protein may be expressed in a form containing a signal peptide. The target protein may contain a signal peptide at the N-terminus thereof. The signal peptide may be removed after expression (e.g., during translocation or localization of the target protein). That is, for example, the target protein to be extracted and the target protein finally obtained may not have the signal peptide.

The signal peptide may be, for example, a peptide having an amino acid sequence of any of the signal peptides exemplified above (e.g., the amino acid sequences of the signal peptides of various organisms such as the amino acid sequence shown as SEQ ID NO: 5). The signal peptide may also be, for example, a variant of any of the signal peptides exemplified above (e.g., a variant of a peptide having an amino acid sequence of any of signal peptides of various organisms such as the amino acid sequence shown as SEQ ID NO: 5), as long as the original function is maintained. For the variant of signal peptide, the description for variants (e.g., conservative variants) of ERp27 and TMX1 mentioned later can be applied. The variant of signal peptide may be, for example, a peptide having an amino acid sequence of signal peptides exemplified above (e.g., amino acid sequences of signal peptides of various organisms such as the amino acid sequence shown as SEQ ID NO: 5) but which includes substitution, deletion, insertion, and/or addition of one or several amino acid residues at one or several positions. The term "one or several" mentioned above in the definition of variants of signal peptides may specifically mean, for example, 1 to 5, preferably 1 to 4, more preferably 1 to 3, even more preferably 1 or 2. The variant of signal peptide may also be, for example, a peptide having an amino acid sequence having preferably at least 50% identity, more preferably at least 60% identity, further preferably at least 70% identity, especially preferably at least 80% identity, to the entire amino acid sequence of any of the signal peptides exemplified above (e.g., the amino acid sequences of the signal peptides of various organisms such as the amino acid sequence shown as SEQ ID NO: 5).

The expression "the original function is maintained" used for the variant of signal peptide may mean that the variant has such a function that the target protein expressed in the plant in a form having the variant added (e.g., added to the N-terminus) is translocated or localized to a specific intracellular organelle or intracellular compartment (e.g., endoplasmic reticulum). This function is also referred to as "function of signal peptide". Whether a certain peptide has the function of signal peptide can be confirmed by, for example, confirming localization of a target protein when the target protein is expressed in plants with the peptide added.

Nucleotide sequence encoding signal peptide is not particularly limited as long as it encodes any of such signal peptides as exemplified above.

Examples of the peptide tag include HA tag, His tag, FLAG tag, GST tag, Myc tag, HSV tag, V5 tag, T7 tag, Strep-tag II, CBD (chitin binding domain), MBP (maltose binding protein), CBP (cellulose binding protein), TRX (thioredoxin), GFP (green fluorescent protein), HRP (horseradish peroxidase), ALP (alkaline phosphatase), and Fc region of antibodies. Examples of the peptide tag include, especially, HA tag, which will be described later. Examples of the His tag include 6xHis tag and 10xHis tag. The peptide tag may be used for, for example, detection or purification of the expressed target protein.

Examples of the protease recognition sequence include recognition sequence of the Factor Xa protease and recognition sequence of the proTEV protease. The recognition sequence of protease can be used for, for example, cleavage of the expressed target protein. Specifically, for example, when the target protein is expressed as a fusion protein with an additional sequence such as a peptide tag, a protease recognition sequence can be inserted in the linkage site of the target protein and the additional sequence, and thereby the target protein not having the additional sequence can be obtained by cleaving the additional sequence from the expressed target protein with a corresponding protease.

The target protein may contain an HA tag at the time of expression. That is, the target protein may be expressed in a form containing an HA tag. Expression of the target protein in a form containing an HA tag may increase the expression amount of the target protein. That is, expression of the target protein in a form containing an HA tag may increase the expression amount of the target protein compared with expression of the target protein in a form not containing the HA tag. The increase of the expression amount of the target protein may be due to, for example, increased transcription of the target protein gene, increased translation of the target protein, reduced degradation of the target protein, or a combination thereof. The target protein may also contain an HA tag at the time of expression and extraction. That is, the target protein may be expressed and extracted in a form containing an HA tag.

Examples of the HA tag include a peptide having the amino acid sequence shown as SEQ ID NO: 17 and a variant thereof. That is, the term "HA tag" shall encompass peptides having the amino acid sequence shown as SEQ ID NO: 17 as well as variants thereof. For variant of the HA tag, the descriptions for variants of ERp27 and TMX1 (e.g., conservative variants) described later can be applied.

The variants of the HA tag are not particularly limited as long as they contain a tyrosine residue and maintain the original function. The variants of HA tag may be, for example, peptides having an amino acid sequence of SEQ ID NO: 17 but which includes substitution, deletion, insertion, and/or addition of one or several amino acids at one or several positions. The term "one or several" mentioned above in the definition of variant of HA tag may specifically mean, for example, 1 to 5, preferably 1 to 4, more preferably 1 to 3, even more preferably 1 or 2. The variant of HA tag may also be, for example, a peptide having an amino acid sequence having preferably at least 50% identity, more preferably at least 60% identity, further preferably at least 70% identity, especially preferably at least 80% identity, to the entire sequence of the amino acid sequence shown as SEQ ID NO: 17. Specific examples of the variant include the HAΔYA tag (YPYDVPD, SEQ ID NO: 18) and the HAΔVPDYA tag (YPYD, SEQ ID NO: 19). That is, the HA tag may have, for example, the amino acid sequence shown as SEQ ID NO: 18 or 19. In addition, amino acid sequence of variant of the HA tag (e.g., an amino acid sequence of SEQ ID NO: 17 but which includes substitution, deletion, insertion, and/or addition of one or several amino acid residues at one or several positions, or an amino acid sequence having such identity as defined above to the entire sequence of the amino acid sequence shown as SEQ ID NO: 17) may preferably be configured so that, for example, it has the amino acid sequence shown as SEQ ID NO: 18 or 19 (especially SEQ ID NO: 19), that is, the amino acid sequence shown as SEQ ID NO: 18 or 19 (especially SEQ ID NO: 19) is conserved.

The number of the tyrosine residues contained in the HA tag is not particularly limited, as long as the purpose of the present invention is not impaired. The number of tyrosine residues contained in the HA tag may be, for example, 1 or larger, 2 or larger, or 3 or larger, and 10 or smaller, 7 or smaller, 5 or smaller, 4 or smaller, 3 or smaller, or 2 or smaller, or in any range defined by a non-conflicting combination of these exemplary minimum and maximum numbers. The number of tyrosine residues contained in the HA tag may be, for example, 1 to 10, 1 to 5, 2 to 10, 2 to 5, 2 to 4, 2 to 3, 3, or 2.

The expression that "the original function is maintained" for variant of the HA tag may mean that the variant has a function of increasing the expression amount of the target protein in the plant, when the target protein is expressed with the variant added. This function is also referred to as the "function of HA tag". Whether a certain peptide has the function of HA tag can be confirmed by, for example, confirming that the expression amount of the target protein obtained when it is expressed in the plant with the peptide added is larger than that of the target protein when it is expressed in the plant without the peptide added. Expression amount of a protein can be determined by, for example, SDS-PAGE or Western blotting.

Nucleotide sequence encoding HA tag is not particularly limited as long as it encodes any of such HA tags as exemplified above.

The number of HA tags contained in the target protein is not particularly limited as long as the purpose of the present invention is not impaired. The number of HA tags contained in the target protein may be, for example, 1 or larger, 2 or larger, 3 or larger, 4 or larger, or 5 or larger, and 20 or smaller, 15 or smaller, 10 or smaller, 7 or smaller, 5 or smaller, 4 or smaller, 3 or smaller, or 2 or smaller, or in any range defined by a non-conflicting combination of these exemplary minimum and maximum numbers. The number of HA tags contained in the target protein may specifically be, for example, 1 to 10, 1 to 5, 1 to 3, 2 to 4, 3, 2, or 1. The number of HA tags contained in the target protein may be, in particular, 3. If the target protein contains 2 or more HA tags, the amino acid sequences of those HA tags may or may not be identical to each other or one another. If the target protein contains two or more HA tags, the description for HA tag can be independently applied to each of those HA tags.

The position of the HA tag in the target protein is not particularly limited as long as the purpose of the present invention is not impaired. The HA tag may be linked to, for example, the N-terminus, C-terminus, or both of the target protein. The HA tag may be linked to, in particular, at least the N-terminus of the target protein. The HA tag may be linked only to the N-terminus or C-terminus of the target protein in any of the numbers exemplified above, may be linked to the N-terminus and C-terminus of the target protein in any of the numbers exemplified above for each terminus, or may be linked to the N-terminus and C-terminus so that the total number of HA tags is any of the numbers exemplified above. When the target protein contains another additional sequence in addition to the HA tag, the positions of the HA tag and the other additional sequence can be appropriately set according to various conditions, such as the type of the additional sequence other than the HA tag. For example, when the target protein contains an HA tag and a signal peptide at the N-terminus, the signal peptide may be linked on the N-terminus side compared to the HA tag. Further, for example, when the target protein contains an HA tag and another peptide tag at the N-terminus, the peptide tag other than the HA tag may be linked on the N-terminus side compared to the HA tag or the C-terminus side compared to the HA tag.

If the target protein is a multimeric protein, only one of the subunits constituting the target protein may contain an HA tag, or two or more (e.g., all) of the subunits constituting the target protein may contain an HA tag. If the target protein is a multimeric protein, in particular, all of the subunits constituting the target protein may contain an HA tag. If two or more of the subunits constituting the target protein contain an HA tag, the configurations (e.g., amino acid sequences, numbers, and positions) of the HA tags in those subunits may or may not be identical to each other or one another. If two or more of the subunits constituting the target protein contain HA tags, the description for HA tag can be independently applied to each of the HA tags contained in those subunits. When the target protein is a multimeric protein, the expression that "the target protein contains X of HA tags" means that at least one of the subunits constituting the target protein contains X of HA tags, or two or more of the subunits constituting the target protein (for example, all of the subunits) constituting the target protein each contain X of HA tags.

The target protein gene is not particularly limited as long as it encodes such a target protein as exemplified above.

The term "gene" refers to not only a gene consisting of DNA, but also a gene consisting of any polynucleotide, as long as it encodes a corresponding expression product. In other words, the term "target protein gene" may mean any polynucleotide encoding a target protein. The target protein gene may be DNA, RNA, or a combination thereof. The target protein gene may be single-stranded or double-stranded. The target protein gene may be single-stranded DNA or single-stranded RNA. The target protein gene may be double-stranded DNA, double-stranded RNA, or a hybrid strand consisting of DNA and RNA strands. The target protein gene may contain both DNA and RNA residues in a single polynucleotide strand. The target protein gene may or may not contain an intron. The type of the target protein gene may be selected according to various conditions, such as the means for expressing the target protein.

The target protein gene or a component thereof can be obtained by, for example, cloning. Nucleic acids such as genomic DNA and cDNA can be used for cloning. The target protein gene can also be obtained by chemical synthesis (Gene, 60(1), 115-127 (1987)).

The obtained target protein gene or a component thereof can be used as it is or after appropriate modification. In other words, a variant of the target protein gene or a component thereof can be obtained by modifying it. Further, for example, a nucleotide sequence encoding an additional sequence may be added to the target protein gene. The target protein gene or a component thereof can be modified by known methods. For example, a site-directed mutagenesis method can be used to introduce a desired mutation at a target site of DNA. Examples of the site-specific mutagenesis method include methods using PCR (Higuchi, R., 61, in PCR technology, Erlich, H.A. Eds., Stockton press (1989); and Carter, P., Meth. in Enzymol., 154, 382 (1987), and methods using phages (Kramer, W. and Frits, H.J., Meth. in Enzymol., 154, 350 (1987); and Kunkel, T.A. et al., Meth. in Enzymol., 154, 367 (1987)). A variant of the target protein gene or a component thereof may also be obtained directly by chemical synthesis.

A plant having the target protein gene can be obtained, for example, by introducing the gene into a plant. The mode for introducing the target protein gene into the plant is not particularly limited. It is sufficient that the target protein gene is retained in the plant in such a manner that it can be expressed. A structure containing the target protein gene in such a manner that it can be expressed is also referred to as an "expression cassette of the target protein gene". In other words, the plant may have the target protein gene in the form of an expression cassette of the target protein gene. For example, when the target protein gene is introduced into a plant in a form requiring transcription such as DNA, the target protein gene may be retained in such a manner that it can be expressed under the control of a promoter that functions in the plant. In addition, for the expression of the target protein gene, another expression regulatory sequence (e.g., enhancer and terminator) may be used in combination. That is, the expression cassette of the target protein gene may contain, for example, an expression regulatory sequence (e.g., promoter, enhancer, and terminator) at an appropriate position in addition to the target protein gene. In the plant, the target protein gene may be present outside the chromosome or introduced into the chromosome. When 2 or more genes are introduced, it is sufficient that each gene is retained in the plant in such a manner that it can be expressed. Such 2 or more genes may be introduced, for example, separately or together. Such 2 or more genes may be introduced, for example, as an operon. It is sufficient that the plant has the target protein gene until it expresses the target protein to a desired degree. That is, the plant may or may not have the target protein gene after expression of the target protein. For example, the plant may or may not have the target protein gene at the time of extraction of the target protein.

The promoter is not particularly limited as long as it functions in the plant that expresses the target protein. The term "promoter that functions in a plant" means a promoter that shows a promoter activity in the plant. The promoter may be a promoter of host origin or a promoter of heterologous origin. The promoter may be a promoter native to the target protein gene or a promoter of another gene. The promoter may be a stronger promoter than the native promoter of the target protein gene. Examples of promoter that functions in plants include plant-derived promoters and plant virus-derived promoters. Examples of the plant-derived promoters include those listed in the Plant Promoter Database (ppdb). Specific examples of the plant-derived promoters include ubiquitin promoter. Examples of plant virus-derived promoter include DNA promoters derived from DNA viruses such as cauliflower mosaic virus (CaMV), commelina yellow mottle virus (CoYMV), rice tungro bacilliform virus (RTBV), sugarcane bacilliform virus (SCBV), soybean chlorotic mottle virus (SbCMV), figwort mosaic virus (FMV), carnation etched ring virus (CERV), peanut chlorotic streak virus (PCSV), strawberry vein banding virus (SVBV), cacao swollen shoot virus (CSSV), and cassava vein mosaic virus (CsVMV). Examples of promoter derived from cauliflower mosaic virus (CaMV) include the 35S promoter and 19S promoter of CaMV In addition, as the promoter, highly active forms of existing promoters may be obtained by using various reporter genes, and utilized. Examples of methods for evaluating strength of promoters and strong promoters are described in the paper of Goldstein et al. (Prokaryotic promoters in biotechnology. Biotechnol. Annu. Rev., 1, 105-128 (1995)), and so forth.

The target protein gene can be introduced into a plant by using, for example, a vector containing the gene (specifically, a vector containing an expression cassette of the gene). A vector containing a target protein gene is also referred to as "expression vector of a target protein gene". The expression vector of the target protein gene can be constructed by, for example, ligating a nucleic acid fragment (e.g., DNA fragment) containing the target protein gene with a vector. By introducing an expression vector of a target protein gene into a plant, the gene can be introduced into the plant. The vector may have a marker such as a drug resistance gene. The vector may also have an expression regulatory sequence such as promoter, enhancer, and terminator for expressing an inserted gene. When constructing an expression vector of a target protein gene, for example, a pre-constructed expression cassette of the target protein gene may be inserted into a vector, or the target protein gene may be ligated with an expression regulatory sequence contained in a vector to construct an expression cassette of the target protein gene on the vector. The vector can be appropriately selected according to various conditions, such as the type of plant and the mode of introduction of the target protein gene. Examples of vectors that can be used for gene transfer into plants include plasmid vectors and virus vectors. Examples of plasmid vectors include binary vectors such as pRI series vectors (TaKaRa BIO), pCambia series vectors (Cosmo Bio), and pBI series vectors (Thermo Fisher Scientific). Examples of virus vectors include vectors derived from plant viruses. Examples of vectors derived from plant viruses include vectors derived from RNA viruses such as tobacco mosaic virus (TMV) and cowpea mosaic virus (CPMV), and vectors derived from DNA viruses such as those exemplified above. Expression vectors derived from RNA viruses can be prepared by, for example, constructing a DNA vector containing the target protein gene and further reverse transcripting an infectious RNA from it.

The target protein gene can also be introduced into a plant by, for example, introducing a nucleic acid fragment containing the gene (specifically, a nucleic acid fragment containing an expression cassette of the gene) into the plant. Examples of such a nucleic acid fragment include linear DNA and linear RNA.

The method for introducing a nucleic acid such as vector and nucleic acid fragment into a plant can be appropriately selected according to various conditions such as the type of the plant. Examples of the method for introducing a nucleic acid such as vector and nucleic acid fragment into a plant include the *Agrobacterium* method, whisker method, particle gun method, and electroporation method. If the vector is a virus vector, the vector (i.e., virus) can be introduced into the plant by infecting the plant with the vector.

For example, a plant that have been subjected to a treatment for introducing the target protein gene may be used as it is for expression of the target protein as a plant having the target protein gene. Alternatively, for example, a plant of a descendant generation having the target protein gene may be obtained from a plant subjected to a treatment for introducing the target protein gene, and the plant of a descendant generation may be used for expression of the target protein as a plant having the target protein gene.

The description for the introduction of a target protein gene can be applied to the introduction of any other gene (e.g., ERp27 gene and/or TMX1 gene).

The plant of the present invention is a plant that has been modified to express ERp27 and/or TMX1. In other words, the plant of the present invention is a plant that has been modified to have the ERp27 gene and/or the TMX1 gene. That is, the plant of the present invention is also a plant having the target protein gene and the ERp27 and/or TMX1 gene. The plant of the present invention is also a plant that expresses the target protein as well as ERp27 and/or TMX1.

The plant of the present invention may have been modified to express only one of ERp27 and TMX1 or both ERp27 and TMX1. In either case, the target protein may be expressed in a form containing a signal peptide or not containing any signal peptide. The target protein may be expressed in a form containing a signal peptide, for example, when the plant has been modified to express TMX1. The target protein may also be expressed in a form containing a signal peptide, for example, when the plant has been modified to express only TMX1 (i.e., to express only TMX1 among ERp27 and TMX1).

Such a modification of a plant that the plant comes to express ERp27 and/or TMX1 can be achieved by introducing the ERp27 and/or TMX1 gene into the plant. The ERp27 and/or TMX1 gene may be retained by the plant of the present invention in such a manner that it can be expressed. The description for the introduction of target protein gene can be applied to the introduction of the ERp27 and/or TMX1 gene. The modifications to construct the plant of the present invention can be carried out in any order. That is, the target protein gene can be introduced into the plant before further introducing the ERp27 gene and/or TMX1 gene, the ERp27 gene and/or the TMX1 gene can be introduced into the plant before further introducing the target protein gene, or the target protein gene and the ERp 27 gene and/or TMX1 gene may also be introduced simultaneously.

By modifying a plant so as to express ERp27 and/or TMX1, the ability of the plant to produce the target protein can be increased, i.e., target protein production by the plant can be increased. Examples of the "increase of target protein production" include increase of accumulation amount of the target protein within or outside the plant.

ERp27 and TMX1 are both proteins that can be classified into the protein disulfide isomerase (PDI) family. ERp27 is also called "endoplasmic reticulum resident protein 27", "FLJ32115", "PDIA8," etc. The gene encoding ERp27 is also referred to as "ERp27 gene". The function of ERp27 is not known. TMX1 is also referred to as "thioredoxin-related transmembrane protein 1," "TMX", "PDIA11" etc. The gene encoding TMX1 is also referred to as "TMX1 gene". TMX1 can have an activity to catalyze reactions that form, cleave, and rearrange disulfide bonds in proteins. This activity is also referred to as "protein disulfide isomerase activity (PDI activity)". Examples of ERp27 and TMX1 and genes encoding them include those of various organisms such as mammals. Examples of mammals include primates such as human, monkey, and chimpanzee, rodents such as mouse, rat, hamster, and guinea pig; and other various mammals such as rabbit, horse, cattle, sheep, goat, pig, dog, and cat. Examples of mammals include, especially, human. The nucleotide sequences of the ERp27 and TMX1 genes derived from various organisms and the amino acid sequences of ERp27 and TMX1 encoded by the genes can be obtained from, for example, public databases such as NCBI and technical literature such as patent documents. The nucleotide sequence of the human ERp27 gene and the amino acid sequence of ERp27 encoded by the gene are shown as SEQ ID NOS: 20 and 21, respectively. The nucleotide sequence of the human TMX1 gene and the amino acid sequence of TMX1 encoded by the gene are shown as SEQ ID NOS: 22 and 23, respectively.

The ERp27 gene may be, for example, a gene having any of the nucleotide sequences of the ERp27 gene exemplified above (e.g., the nucleotide sequences of the ERp27 genes of various organisms such as the nucleotide sequence shown as SEQ ID NO: 20). ERp27 may be, for example, a protein having any of the amino acid sequences of ERp27 exemplified above (e.g., the amino acid sequences of ERp27 of various organisms such as the amino acid sequence shown as SEQ ID NO: 21). The TMX1 gene may be, for example, a gene having any of the nucleotide sequences of the TMX1 gene exemplified above (e.g., the nucleotide sequences of the TMX1 gene of various organisms such as the nucleotide sequence shown as SEQ ID NO: 22). TMX1 may be, for example, a protein having any of the amino acid sequences of TMX1 exemplified above (e.g., the amino acid sequences of TMX1 of various organisms such as the amino acid sequence shown as SEQ ID NO: 23). The expression "a gene has a nucleotide sequence" may mean that the gene contains the nucleotide sequence, and may also mean that the gene consists of the nucleotide sequence, unless otherwise specified. The expression "a protein has an amino acid sequence" may mean that the protein contains the amino acid sequence, and may also mean that the protein consists of the amino acid sequence, unless otherwise specified.

The ERp27 gene and TMX1 gene may be variants of any of the ERp27 genes and TMX1 genes exemplified above, respectively, as long as the original functions thereof are maintained (e.g., variants of genes having the nucleotide sequences of the ERp27 gene of various organisms such as the nucleotide sequence shown as SEQ ID NO: 20 for the ERp27 gene, and variants of the genes having the nucleotide sequences of the TMX1 gene of various organisms such as the nucleotide sequence shown as SEQ ID NO: 22 for the TMX1 gene). Similarly, ERp27 and TMX1 may be variants of ERp27 and TMX1 exemplified above, respectively (e.g., variants of proteins having the amino acid sequence of ERp27 of various organisms such as the amino acid sequence shown as SEQ ID NO: 21 for ERp27, and variants of proteins having the amino acid sequence of TMX1 of various organisms such as the amino acid sequence shown as SEQ ID NO: 23 for TMX1), as long as the original functions thereof are maintained. Such a variant that maintains the original function may be referred to as "conservative variant". The terms "ERp27 gene" and "TMX1 gene" shall encompass the ERp27 genes and TMX1 genes exemplified above, respectively, as well as conserved variants thereof. Similarly, the terms "ERp27" and "TMX1" shall encompass the ERp27 and TMX1 exemplified above, respectively, as well as conserved variants thereof. Examples of such conservative variants include, for example, homologues and artificial modifications of the ERp27 and TMX1 genes as well as ERp27 and TMX1 exemplified above.

The expression "original function is maintained" means that a variant of the gene or protein has a function (e.g., activity or property) that corresponds to the function (e.g., activity or property) of the original gene or protein. In other words, the expression "original function is maintained" for a gene may mean that a variant of the gene encodes a protein that maintains the original function. That is, the expression "original function is maintained" for the ERp27 and TMX1 genes may mean that variants of the genes encode ERp27 and TMX1, respectively. Further, the expression "original function is maintained" for ERp27 and TMX1 may mean that variants of the proteins have functions as ERp27 and TMX1, respectively. Both "function as ERp27" and "function as TMX1" may mean, for example, a function of increasing target protein production by a plant when expressed in the plant. Further, "function as TMX1" may mean, for example, the PDI activity.

Whether a variant of a protein has a function of increasing target protein production by a plant when expressed in the plant can be confirmed by confirming increase in target protein production by the plant in which the variant is expressed.

The PDI activity can be measured by incubating the enzyme with a substrate (e.g., protein) and measuring modification (e.g., formation, cleavage, and/or rearrangement) of disulfide bonds in the substrate.

Examples of the conservative variant will be explained below.

Homologues of the ERp27 and TMX1 genes or homologues of ERp27 and TMX1 can be easily obtained from public databases by, for example, BLAST search or FASTA search using any of the nucleotide sequences of the ERp27 and TMX1 genes exemplified above or the amino acid sequences of ERp27 and TMX1 exemplified above as a query sequence. Homologues of the ERp27 and TMX1 genes can also be obtained by, for example, PCR using any of the chromosomes of various organisms as the template and oligonucleotides prepared on the basis of these known nucleotide sequences of ERp27 and TMX1 genes as primers.

Both the ERp27 gene and the TMX1 gene may be a gene encoding a protein having an amino acid sequence derived from any of the amino acid sequences mentioned above (e.g., the amino acid sequences of ERp27 of various organisms such as the amino acid sequence shown as SEQ ID NO: 21 for ERp27, and the amino acid sequences of TMX 1 of various organisms such as the amino acid sequence shown as SEQ ID NO: 23 for TMX 1) by substitution, deletion, insertion or addition of one or several amino acids at one or several positions. For example, the encoded protein may have an extended or shortened N- and/or C-terminus. The term "one or several" mentioned above means, depending on the position and type of amino acid residues in the three-dimensional structure of the protein, specifically, for example, 1 to 50, 1 to 40, or 1 to 30, preferably 1 to 20, more preferably 1 to 10, further preferably 1 to 5, especially preferably 1 to 3.

The substitution, deletion, insertion, and/or addition of one or several amino acids mentioned above is a conservative mutation that maintain the normal function of the protein. Typical examples of the conservative mutation include conservative substitutions. Conservative substitutions are such mutations that mutual substitution occurs among Phe, Trp, and Tyr when the substitution site is an aromatic amino acid, among Leu, Ile, and Val when the substitution site is a hydrophobic amino acid, between Gln and Asn when the substitution site is a polar amino acid, among Lys, Arg, and His when the substitution site is a basic amino acid, between Asp and Glu when the substitution site is an acidic amino acids, and between Ser and Thr when the substitution site is an amino acid with hydroxyl group. Specific examples of substitutions considered conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned substitution, deletion, insertion, or addition of amino acids may be those resulting from a naturally-occurring mutation due to an individual difference, difference of species, or the like of the organism from which the gene is derived (mutant or variant).

The ERp27 and TMX1 genes may also be a gene encoding a protein having an amino acid sequence showing, for example, at least 50% identity, at least 65% identity, or at least 80% identity, preferably at least 90% identity, more preferably at least 95% identity, further preferably at least 97% identity, especially preferably at least 99% identity, to the entire sequence of any of the amino acid sequences mentioned above, as long as the original function is maintained.

Both the ERp27 gene and the TMX1 gene may also be a gene (e.g., DNA) that hybridizes to a probe that can be prepared from any of the nucleotide sequences mentioned above (e.g., the nucleotide sequences of ERp27 gene of various organisms such as the nucleotide sequence shown as SEQ ID NO: 20 for the ERp27 gene, and the nucleotide sequences of TMX1 gene of various organisms such as the nucleotide sequence shown as SEQ ID NO: 22 for the TMX 1 gene), such as a sequence complementary to the entire sequence of any of the aforementioned nucleotide sequences or a part thereof, under stringent conditions, as long as the original function is maintained. The term "stringent conditions" refers to conditions under which a so-called specific hybrid is formed, and a non-specific hybrid is not formed. Examples of the stringent conditions include those under which DNAs having a high identity to each other, for example, DNAs having at least 50% identity, at least 65% identity, or at least 80% identity, preferably at least 90% identity, more preferably at least 95% identity, further preferably at least 97% identity, particularly preferably at least 99% identity, to each other hybridize to each other, and DNAs having an identity lower than the above do not hybridize to each other, for example, the washing conditions of usual Southern hybridization, i.e., washing once, preferably 2 or 3 times, at salt concentrations and temperature corresponding to 1 x SSC and 0.1% SDS at 60°C, preferably 0.1 x SSC and 0.1% SDS at 60°C, more preferably 0.1 x SSC and 0.1% SDS at 68°C.

As described above, the probe used for the hybridization mentioned above may be a part of a complementary sequence of the gene. Such a probe can be prepared by PCR using oligonucleotides prepared on the basis of a known gene sequence as primers and a DNA fragment containing the gene described above as a template. For example, a DNA fragment of about 300 bp in length can be used as the probe. When a DNA fragment having a length of about 300 bp is used as the probe, the washing conditions of the hybridization can be, for example, 50°C, 2 x SSC, and 0.1% SDS.

In addition, since the degeneracy of codons varies from host to host, both the ERp27 gene and the TMX1 gene may have replacement of any codon with an equivalent codon. In other words, the ERp27 gene and TMX1 gene may be variants of any of the ERp27 genes and TMX1 genes exemplified above occurring due to the degeneracy of codons, respectively. For example, both the ERp27 gene and the TMX1 gene may be modified to have optimal codons according to the codon usage frequency of the host used.

Both ERp27 and TMX1 may contain an additional sequence in addition to any of the amino acid sequences of ERp27 and TMX1 exemplified above. In other words, both the ERp27 and TMX1 genes may contain a nucleotide sequence encoding an additional sequence in addition to any of the nucleotide sequences of the ERp27 and TMX1 gene exemplified above. For the additional sequence, the description for the additional sequence for the target protein can be applied. Both ERp27 and TMX1 may be expressed, for example, in a form containing an additional sequence.

The term "identity" between amino acid sequences means an identity between amino acid sequences calculated with blastp using the default settings of Scoring Parameters (Matrix, BLOSUM62; Gap Costs, Existence=11 and Extension=1; and Compositional Adjustments, Conditional compositional score matrix adjustment). The term "identity" between nucleotide sequences means an identity between nucleotide sequences calculated with blastn using the default settings of Scoring Parameters (Match/Mismatch Scores=1, -2; and Gap Costs=Linear).

The above descriptions for conservative variants of genes and proteins can be applied to any gene and protein.

The plant of the present invention may have any characteristics as long as it can produce the target protein.

The plant of the present invention may have been modified, for example, to express a chaperone. In other words, the plant of the present invention may have been modified to have a chaperone gene. The term "chaperone gene" means a gene encoding a chaperone.

A plant can be modified to express a chaperone by introducing a chaperone gene into the plant. It is sufficient that the chaperone gene is retained in the plant of the present invention in such a manner that it can be expressed. For the introduction of the chaperone gene, the description for the introduction of the target protein gene can be applied. The modifications for constructing the plant of the present invention can be carried out in any order.

The term "chaperone" may refer to a protein that has a function of assisting folding of proteins. This function is also referred to as "function of chaperone". Examples of chaperone include glycosyltransferase. The term "glycosyltransferase" may mean a protein that has an activity to catalyze a reaction to add a sugar chain to a protein. This activity is also referred to as "glycosyltransferase activity". Examples of glycosyltransferase include O-fucosyltransferase. The term "O-fucosyltransferase" may refer to a protein that has an activity to catalyze a reaction to O-fucosylate proteins (i.e., add an O-fucose type sugar chain to proteins). This activity is also referred to as "O-fucosyltransferase activity". Examples of O-fucosyltransferase include O-fucosyltransferase 1. The O-fucosyltransferase 1 may have an activity to catalyze the reaction to O-fucosylate the S/T residue (i.e., Ser or Thr residue) of CX₄₋₅(S/T)C sequence (X is independently any amino acid residue) in a protein. This activity is also referred to as "O-fucosyltransferase 1 activity". Examples of the CX₄₋₅(S/T)C sequence include, especially, CXXXX(S/T)C sequence (X is independently any amino acid residue). The CX₄₋₅(S/T)C sequence may be present in, for example, the epidermal growth factor domain (EGF domain) of a protein. The term "EGF domain" may mean a motif structure consisting of 30 to 40 amino acid residues, including 6 conserved Cys residues forming 3 disulfide bonds. Examples of the EGF domain and CX₄₋₅(S/T)C sequence include those described in Okajima T., Specific O-glycosylation modifying epidermal growth factor domains, SEIKAGAKU (Journal of Japanese Biochemical Society), Vol. 83, No. 9, pp. 813-821, 2011. Examples of the EGF domain and CX₄₋₅(S/T)C sequence also include those contained in DLL4-Fc. Examples of chaperone such as O-fucosyltransferase 1 and chaperone gene such as O-fucosyltransferase 1 gene include those of various organisms such as mammals and insects. Examples of mammals include primates such as human, monkey, and chimpanzee, rodents such as mouse, rat, hamster, and guinea pig; and other various mammals such as rabbit, horse, cattle, sheep, goat, pig, dog, and cat. Examples of mammals include, especially, human. Examples of insects include drosophila. Specific examples of O-fucosyltransferase 1 include human POFUT1 and drosophila OFUT1. Nucleotide sequences of chaperone genes derived from various organisms and amino acid sequences of the chaperones encoded by the genes can be obtained from, for example, public databases such as NCBI or technical literature such as patent documents. The nucleotide sequence of the human POFUT1 gene (the part encoding the mature protein) and the amino acid sequence of POFUT1 (mature protein) encoded by the gene are shown as SEQ ID NOS: 24 and 25, respectively.

The chaperone gene may be, for example, a gene having a nucleotide sequence of any of the chaperone genes exemplified above (e.g., the nucleotide sequences of the chaperone genes of various organisms, such as the nucleotide sequence shown as SEQ ID NO: 24). The chaperone may be, for example, a protein having an amino acid sequence of any of the chaperones exemplified above (e.g., the amino acid sequences of chaperones of various organisms such as the amino acid sequence shown as SEQ ID NO: 25). The chaperone gene may also be, for example, a conservative variant of any of the chaperone genes exemplified above. Similarly, the chaperone may be, for example, a conservative variant of any of the chaperones exemplified above. The variants are not particularly limited as long as the original function thereof is maintained. For conserved variants of chaperone and chaperone gene, the descriptions for conserved variants of ERp27 and TMX1 and the genes encoding them can be applied. For example, the chaperone gene (e.g., O-fucosyltransferase 1 gene) may be a gene encoding a protein having an amino acid sequence derived from any of the amino acid sequences of the chaperones exemplified above (e.g., amino acid sequences of chaperones of various organisms such as the amino acid sequence shown as SEQ ID NO: 25) by substitution, deletion, insertion or addition of one or several amino acids at one or several positions. The chaperone gene (e.g., O-fucosyltransferase 1 gene) may also be, for example, a gene encoding a protein having an amino acid sequence having at least 80% identity, preferably at least 90% identity, more preferably at least 95% identity, further preferably at least 97% identity, particularly preferably at least 99% identity, to the entire amino acid sequence of any of the chaperones exemplified above (e.g., amino acid sequences of chaperones of various organisms such as the amino acid sequence shown as SEQ ID NO: 25). In the case of a chaperone, the expression "the original function is maintained" may mean that a variant of the protein has the function of the chaperone. In the case of glycosyltransferase, for example, the expression "the original function is maintained" may mean that a variant of the protein has the glycosyltransferase activity. In the case of O-fucosyltransferase, for example, the expression "the original function is maintained" may mean that a variant of the protein has the O-fucosyltransferase activity. In the case of O-fucosyltransferase 1, for example, the expression "the original function is maintained" may mean that a variant of the protein has the O-fucosyltransferase 1 activity. Function of chaperone or activity of each enzyme can be measured by, for example, conventional methods. For example, the O-fucosyltransferase 1 activity can be measured by incubating the enzyme with a substrate (e.g., GDP-fucose and proteins to be O-fucosylated) and measuring enzyme-dependent O-fucosylation of the protein.

Expression of a chaperone may improve, for example, production of the target protein. Expression of a chaperone may also improve, for example, folding of the target protein. Such improved folding of the target protein may, for example, reduce aggregation of the target protein. Further, if the target protein can form a multimer, the improved folding of the target protein may, for example, promote formation of the multimer of the target protein. Specifically, expression of a chaperone may improve, for example, folding of the target protein, and thereby improve production of the target protein.

In one embodiment, expression of O-fucosyltransferase 1 may improve, for example, production of the target protein having the CX₄₋₅(S/T)C sequence. In another embodiment, expression of O-fucosyltransferase 1 may improve, for example, folding of the target protein having the CX₄₋₅(S/T)C sequence. In another embodiment, expression of O-fucosyltransferase 1 may specifically improve, for example, folding of the target protein having the CX₄₋₅(S/T)C sequence, and thereby improve production of the target protein. That is, O-fucosylation of the S/T residues of the CX₄₋₅(S/T)C sequence in the target protein may improve folding of the target protein. For example, folding of the target protein may be improved by O-fucosylation of S/T residues of the CX₄₋₅(S/T)C sequence, especially when the target protein has two or more EGF domains containing the CX₄₋₅(S/T)C sequence. That is, the target protein may have, for example, the CX₄₋₅(S/T)C sequence. The target protein may have the CX₄₋₅(S/T)C sequence, for example, in the EGF domain, i.e., it may have an EGF domain containing the CX₄₋₅(S/T)C sequence. The target protein may have, for example, 2 or more EGF domains containing the CX₄₋₅(S/T)C sequence. For example, a Notch ligand such as DLL4-Fc may be a protein having the CX₄₋₅(S/T)C sequence. For example, a Notch ligand such as DLL4-Fc can be a protein having 2 two or more EGF domains containing the CX₄₋ₛ(S/T)C sequence.

### <2> Expression of target protein

By culturing the plant of the present invention, the target protein can be expressed, and thereby the target protein can be accumulated in or outside the plant. The "culture of plant" is not limited to cell culture or tissue culture, but also includes so-called "cultivation", such as cultivation of the whole plant body. By the culture, the plant may or may not, for example, propagate, grow, and/or differentiate.

The culture conditions are not particularly limited as long as the target protein is expressed. The culture conditions can be appropriately set, for example, according to various conditions, such as the type of plant. The culture can be performed, for example, under the usual conditions used for plant culture. The culture may be performed by using, for example, liquid medium or soil. The liquid medium or soil may contain, for example, components useful for plant culture, such as fertilizers. If necessary, expression of the target protein may be induced. The culture may be performed, for example, under light conditions, dark conditions, or a combination thereof. The culture temperature may be, for example, 5 to 40°C, 10 to 35°C, or 15 to 30°C. The culture may be performed, for example, until the target protein is expressed to a desired degree. The culture period may be, for example, 1 hour or longer, 3 hours or longer, 6 hours or longer, 12 hours or longer, 1 day or longer, 2 days or longer, 3 days or longer, 4 days or longer, 5 days or longer, 6 days or longer, or 7 days or longer, and 30 days or shorter, 20 days or shorter, 15 days or shorter less, 10 days or shorter, 70 days or shorter, or 5 days or less, or in a range defined by a non-conflicting combination of these minimum and maximum periods. The culture period may specifically be, for example, 4 to 10 days.

### <3> Collection of target protein

The target protein may be collected as appropriate. That is, the method of the present invention may further comprise a step for collecting the target protein. This step is also referred to as "collection step".

For example, if the target protein accumulates in the plant, the target protein can be extracted from the plant. That is, the collection step may include, for example, a step for extracting the target protein from the plant. The step is also referred to as "extraction step".

The extraction step may be carried out, for example, in the presence of an antioxidant and/or metal sequestering agent. The antioxidant and/or metal sequestering agent is also referred to as "active ingredient". By carrying out the extraction step in the presence of the active ingredient, formation of a byproduct derived from the target protein may be reduced. That is, by utilizing the active ingredient, generation of a byproduct derived from the target protein in the extraction step may be reduced compared with the case where the active ingredient is not utilized. The byproduct derived from the target protein is also referred to simply as "byproduct".

The term "byproduct derived from the target protein" may mean an unintended product derived from the target protein. The byproduct may be derived from the target protein in its entirety or from a part of the target protein. For example, if the target protein is a multimeric protein, the byproduct may be derived from at least one or at least one kind of the multiple subunits or multiple kinds of subunits constituting the multimeric protein, or it may be derived from all of those subunits. Examples of the byproduct include products derived from the target protein in which an unintended covalent bond is formed. The unintended covalent bond may be formed, for example, within and/or between target protein molecules. When the target protein is a multimeric protein, the expression that "a covalent bond is formed within the target protein molecule" also means that the covalent bond is formed between subunits within the target protein molecule. Examples of the unintended covalent bond include dityrosine cross-link. Examples of the dityrosine cross-link include those formed between tyrosine residues present in the HA tag contained in the target protein. Specific examples of the byproduct include an analogue of the target protein and aggregate of the target protein. The term "analogue of the target protein" may mean a byproduct derived from the target protein that has been generated by formation of an unintended covalent bond within the target protein molecule. Examples of the analogue of the target protein includes, when the target protein is a multimeric protein, a product resulting from bonding of two or more subunits within a multimeric protein molecule with an unintended covalent bond, such as dityrosine cross-link. The term "aggregate of target protein" may refer to a byproduct derived from the target protein and that has been generated by formation of an unintended covalent bond between target protein molecules. Examples of the aggregate of the target protein include a product resulting from bonding of two or more target protein molecules with unintended covalent bonds such as dityrosine cross-link. The byproduct may be, for example, a soluble or precipitating substance.

Examples of the reduction of byproduct production include reduction of byproduct production amount per unit dry weight of the plant, reduction of the ratio of byproduct production amount to target protein expression amount, and reduction of the ratio of byproduct production amount to yield of the target protein.

Reduction of the production of the byproduct during extraction may improve, for example, production of the target protein. Examples of the improvement of production of the target protein include increase in yield of the target protein per unit dry weight of the plant, increase in ratio of yield of the target protein to expression amount of the target protein, and increase in ratio of yield of the target protein to production amount of the byproduct.

The plant used in the extraction step is not particularly limited as long as it contains the target protein. For the extraction step, the whole plant body or a part of the plant body may be used. For the extraction step, the whole plant obtained by the culture or a part of the plant obtained by the culture may be used. For example, the whole plant body may be cultured to obtain a plant containing the target protein, and a part of the plant (e.g., leaves) may be separated and used for the extraction step.

The term "antioxidant" may mean an ingredient capable of directly or indirectly suppressing oxidation of an object. The antioxidant is not particularly limited so long as it can reduce the generation of byproducts. Reduction of the generation of byproducts may be caused by, for example, decomposition of hydrogen peroxide present in the extraction system by the use of the antioxidant. That is, the antioxidant may be, for example, one that contributes to removal of hydrogen peroxide. Examples of the antioxidant include ascorbic acid, isoascorbic acid, sulfurous acid, tocopherol, ethoxyquin, cysteine, γ-glutamylcysteine, reduced glutathione, manganese dioxide, and catalase. All of these ingredients may contribute to the removal of hydrogen peroxide. Examples of ascorbic acid include L-ascorbic acid. Examples of the antioxidant include, especially, ascorbic acid such as L-ascorbic acid, isoascorbic acid, and sulfurous acid. One type of ingredient may be used, or a combination of two or more types of ingredients may be used as the antioxidant.

The term "metal sequestering agent" may refer to any ingredient capable of sequestering metal ions. The metal sequestering agent is also referred to as "chelating agent". The metal sequestering agent is not particularly limited as long as it can reduce the generation of byproducts. Examples of the metal sequestering agent include EDTA (ethylene diamine tetraacetic acid), NTA (nitrilo triacetic acid), DTPA (diethylene triamine pentaacetic acid), HEDTA (hydroxyethyl ethylene diamine triacetic acid), TTHA (triethylene tetramine hexaacetic acid), PDTA (1,3-propanediamine tetraacetic acid), DPTA-OH (1,3-diamino-2-hydroxypropane tetraacetic acid), HIDA (hydroxyethyl imino diacetic acid), DHEG (dihydroxyethyl glycine), GEDTA (glycol ether diamine tetraacetic acid), CMGA (dicarboxymethyl glutamic acid), EDDS ((S,S)-ethylene diamine disuccinic acid), HEDP (hydroxyethylidene diphosphonic acid), NTMP (nitrilotris (methylene phosphonic acid)), PBTC (phosphonobutane tricarboxylic acid), and EDTMP (ethylene diamine tetra(methylene phosphonic acid)). Examples of the metal sequestering agent include, especially, EDTA. As the metal sequestering agent, one type of ingredient may be used, or a combination of two or more types of ingredients may be used.

Any of ingredients that can form a salt may be used as a free compound, salt, or a combination thereof. In other words, the term "active ingredient" may mean an active ingredient in the form of free compound, salt thereof, or a combination thereof, unless otherwise noted. Specifically, for example, the term "ascorbic acid" may mean free ascorbic acid, a salt thereof, or a combination thereof, unless otherwise noted. All of these ingredients (e.g., compounds in free form and salts) may also be anhydride or hydrate, unless otherwise noted. The salt is not particularly limited as long as it can reduce the generation of byproducts. For example, examples of salts for acidic groups such as carboxyl group include ammonium salts, salts with alkali metals such as sodium and potassium, salts with alkaline earth metals such as calcium and magnesium, aluminum salts, zinc salts, salts with organic amines such as triethylamine, ethanolamine, morpholine, pyrrolidine, piperidine, piperazine, and dicyclohexylamine, and salts with basic amino acids such as arginine and lysine. Examples of salts for basic groups such as amino group include salts with inorganic acids such as hydrochloric acid, sulfuric acid, phosphoric acid, nitric acid, and hydrobromic acid, salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, theoclic acid, salicylic acid, lactic acid, oxalic acid, mandelic acid, malic acid, and methylmalonic acid, and salts with organic sulfonic acids such as methanesulfonic acid, benzenesulfonic acid, and p-toluenesulfonic acid. Specifically, for example, examples of the salts of ascorbic acid include, especially, sodium ascorbate, potassium ascorbate, and calcium ascorbate. Specifically, for example, examples of the salts of sulfurous acid include, especially, sodium sulfite. As the salt, one type of salt may be used, or a combination of two or more types of salts may be used.

The concentration for use of the active ingredient is not particularly limited as long as the purpose of the present invention is not impaired. The concentration for use of the active ingredient may be set so that, for example, the generation of byproducts can be reduced.

The concentration for use of the active ingredient may be, for example, 1 mM or higher, 3 mM or higher, 5 mM or higher, 7 mM or higher, 10 mM or higher, 15 mM or higher, 20 mM or higher, 25 mM or higher, 30 mM or higher, 40 mM or higher, 50 mM or higher, 60 mM or higher, 70 mM or higher, 80 mM or higher, 90 mM or higher, 100 mM or higher, 120 mM or higher, 150 mM or higher, 200 mM or higher, 250 mM or higher, 300 mM or higher, 350 mM or higher, 400 mM or higher, 450 mM or higher, or 500 mM or higher, and may be 1500 mM or lower, 1200 mM or lower, 1000 mM or lower, 900 mM or lower, 800 mM or lower, 700 mM or lower, 600 mM or lower, 500 mM or lower, 450 mM or lower, 400 mM or lower, 350 mM or lower, 300 mM or lower, 250 mM or lower, 200 mM or lower, 150 mM or lower, 140 mM or lower, 130 mM or lower, 120 mM or lower, 110 mM or lower, 100 mM or lower, 90 mM or lower, 80 mM or lower, 70 mM or lower , 60 mM or lower, or 50 mM or lower, or in any range defined by a non-conflicting combination of these minimum and maximum concentrations. The concentration for use of the active ingredient may be, for example, 25 to 1500 mM, 25 to 1000 mM, 25 to 700 mM, 25 to 500 mM, 25 to 300 mM, 25 to 150 mM, 50 to 150 mM, 60 to 140 mM, 70 to 130 mM, 80 to 120 mM, 90 to 110 mM, 100 to 300 mM, 200 to 400 mM, 300 to 500 mM, 400 to 600 mM, 1 to 150 mM, 3 to 100 mM, or 5 to 50 mM. The term "concentration for use of active ingredient" may mean the concentration of the active ingredient in the extraction system (excluding the plant itself). The term "concentration for use of active ingredient" may specifically mean the concentration of the active ingredient in the liquid (excluding the plant itself) in which the plant is disrupted. The term "concentration of active ingredient" may mean, when two or more types of ingredients are selected as the active ingredient, the total concentration of those ingredients, unless otherwise noted.

When the active ingredient contains an antioxidant (i.e., at least an antioxidant is selected as the active ingredient), the concentration for use of the antioxidant may be, for example, in the range of the concentration for use of the active ingredient exemplified above. If the active ingredient contains an antioxidant, the concentration for use of the antioxidant may be, for example, especially, 25 mM or higher, 30 mM or higher, 40 mM or higher, 50 mM or higher, 60 mM or higher, 70 mM or higher, 80 mM or higher, 90 mM or higher, 100 mM or higher, 120 mM or higher, or 150 mM or higher. When the active ingredient contains an antioxidant, the concentration for use of antioxidant may be specifically, for example, 25 to 1500 mM, 25 to 1000 mM, 25 to 700 mM, 25 to 500 mM, 25 to 300 mM, 25 to 150 mM, 50 to 150 mM, 60 to 140 mM, 70 to 130 mM, 80 to 120 mM, 90 to 110 mM, 100 to 300 mM, 200 to 400 mM, 300 to 500 mM, or 400 to 600 mM. When two or more types of ingredients are selected as the antioxidant, "concentration of antioxidant" may mean the total concentration of those ingredients, unless otherwise noted.

When the active ingredient contains a metal sequestering agent (i.e., at least a metal sequestering agent is selected as the active ingredient), the concentration for use of the metal sequestering agent may be, for example, in the range of the concentration for use of the active ingredient exemplified above. When the active ingredient contains a metal sequestering agent, the concentration for use of the metal sequestering agent may be, for example, especially, 1 mM or higher, 3 mM or higher, 5 mM or higher, 7 mM or higher, 10 mM or higher, 15 mM or higher, 20 mM or higher, 25 mM or higher, 30 mM or higher, 40 mM or higher or 50 mM or higher. When the active ingredient contains a metal sequestering agent, the concentration for use of the metal sequestering agent may specifically be, for example, 1 to 150 mM, 3 to 100 mM, or 5 to 50 mM. When two or more types of ingredients are selected as the metal sequestering agent, "concentration of metal sequestering agent" may mean the total concentration of those ingredients, unless otherwise noted.

When the active ingredient contains catalase (i.e., at least catalase is selected as the active ingredient), the concentration for use of catalase is, for example, 10 U/mL or higher, 20 U/mL or higher, 50 U/mL or higher, 100 U/mL or higher, 200 U/mL or higher, 500 U/mL or higher, 1000 U/mL or higher, 2000 U/mL or higher, or 5000 U/mL or higher, and 500000 U/mL or lower, 200000 U/mL or lower, 100000 U/mL or lower, 50000 U/mL or lower, 20000 U/mL or lower, 10000 U/mL or lower, 5000 U/mL or lower, 2000 U/mL or lower, 1000 U/mL or lower, or 500 U/mL or lower, and may be in any range defined by a non-contradictory combination of these minimum and maximum concentrations. When the active ingredient contains catalase, the concentration for use of catalase may specifically be, for example, 10 to 100000 U/mL, 100 to 50000 U/mL, or 1000 to 20000 U/mL. The amount of the enzyme that degrades 1 µmol of hydrogen peroxide in 1 minute at pH 7.0 and 25°C is defined as 1 U of the catalase activity.

The time of using the active ingredient is not particularly limited as long as the purpose of the present invention is not impaired. The time of using the active ingredient may be set so that, for example, the generation of byproducts is reduced. The active ingredient may be present in the extraction system during the entire period of the extraction step or only a part of the period of the extraction step. The expression that "extraction is performed in the presence of the active ingredient" does not mean that the active ingredient should be present in the extraction system during the entire period of the extraction step. For example, the active ingredient may or may not be present in the extraction system at the time of starting the extraction step. If the active ingredient is not present in the extraction system at the time of starting the extraction step, the active ingredient is supplied to the extraction system after the start of the extraction step. If the active ingredient is not present in the extraction system at the time of starting the extraction step, for example, the active ingredient may be supplied to the extraction system within 5 seconds, 10 seconds, 15 seconds, 20 seconds, 30 seconds, or 1 minute from the start of the extraction step. The active ingredient may typically be present in the extraction system at the time of starting the extraction. The term "the time of starting the extraction step" may mean, for example, the time at which a treatment of disrupting the plant is started. For example, the active ingredient may be present in the extraction system at the concentration exemplified above during the entire period of the extraction step, or may be present in the extraction system at the concentration exemplified above during only a part of the period of the extraction step. For example, the active ingredient may be present in the extraction system at the concentration exemplified above at the time of starting the extraction step, or may be supplied to the extraction system to be at the concentration exemplified above after starting the extraction step.

The extraction step can be carried out by, for example, usual methods for extracting proteins from plants. In one embodiment, the extraction step can be carried out by, for example, usual methods for extracting proteins from plants, except that the extraction step is carried out in the presence of the active ingredient. The extraction can be carried out by, for example, disrupting the plant (specifically, the cells constituting the plant). Disruption of the plant can be carried out in a liquid such as water or aqueous buffer. The disruption can be performed by, for example, known methods for disrupting cells such as plant cells. Examples of such methods include ultrasonic disruption, dyno-milling, bead disruption, French press disruption, disruption by a mill, freeze-drying and powdering treatment, and lysozyme treatment. One of these methods may be used independently or two or more of them may be used in combination as appropriate.

By carrying out the extraction step as described above, an extract (e.g., disruption product) containing the target protein is obtained.

The target protein may be collected from the extract (e.g., disruption product) as appropriate. That is, the collection step may further comprise a step of collecting the target protein from the extract. The target protein can be specifically collected from the extract as an appropriate fraction containing the target protein. Examples of such a fraction include supernatant of the extract and purified products thereof. The target protein may be purified to a desired level. For example, the supernatant of the extract may be obtained and the target protein may be purified from the supernatant of the extract. The supernatant of the extract may be obtained by, for example, a solid-liquid separation means. Examples of the solid-liquid separation means include filtration and centrifugation. Purification of the target protein can be performed by, for example, known methods used for purification of proteins. Examples of such methods include, for example, ammonium sulfate fractionation, ion exchange chromatography, hydrophobic chromatography, affinity chromatography, gel filtration chromatography, and isoelectric precipitation. One of these methods may be used independently or two or more of them may be used in combination as appropriate.

Further, for example, if the target protein accumulates outside the plant (e.g., in the culture medium), the target protein can be collected in the same manner as that for the collection from extracts.

The target protein may be obtained in a free state or as an immobilized enzyme immobilized on a solid phase such as a resin.

The collected target protein may be formulated in the form of a preparation as appropriate. The form of the preparation is not particularly restricted and can be appropriately set according to various conditions such as the intended use of the target protein. Examples of the form of the preparation include liquid, suspension, powder, tablet, pill, and capsule. For the formulation, for example, pharmacologically acceptable additives such as excipient, binder, disintegrant, lubricant, stabilizer, flavor enhancer, odor enhancer, flavoring agent, diluent, and surfactant can be used.

### Examples

Hereafter, the present invention will be explained more specifically with reference to the following non-limiting examples.

### Example 1: Expression of protein using tobacco plant expressing PDI as host

### 1. Materials and methods

### (1) Cultivation of tobacco plant and preparation for agroinfiltration treatment

Benthamiana (*Nicotiana benthamiana,* hereafter referred to simply as "tobacco") was used as the host for expression of the target protein. Otsuka Liquid Fertilizer Formula A diluted twice was absorbed in a urethane mat, and tobacco seeds were sown. The urethane mat was covered with a plastic wrap and cultivation was performed at 25°C in the light place (16 hours of daylength) for 2 weeks. After confirming development of cotyledons, the wrap was removed, and the urethane mat was divided into smaller pieces, and transplanted on a hydroponic panel. The hydroponic panel was floated on Otsuka Liquid Fertilizer Formula A diluted twice and contained in a vat, and cultivation was further continued for 3 weeks under the conditions of 25°C, light place (16 hours of daylength), and 1,000 ppm of CO₂. The tobacco plants were planted in an artificially lighted hydroponic system (24°C, 16-hour daylength, and 1,000 ppm CO₂) and cultivation was continued for another 2 weeks to obtain tobacco plants with about 12 developed true leaves. The obtained tobacco plants were used for the following infiltration treatment.

### (2) Construction of vector and introduction into Agrobacterium bacterium

DLL4-Fc, human laminin 511E8, and GFP were selected as the target proteins. DLL4-Fc and human laminin 511E8 A were expressed with a signal sequence for translocation to the endoplasmic reticulum, as secretory proteins secreted through the endoplasmic reticulum (ER). GFP was expressed without any signal sequence for translocation to the endoplasmic reticulum.

The amino acid sequence of DLL4-Fc is shown as SEQ ID NO: 1 and the nucleotide sequence encoding DLL4-Fc is shown as SEQ ID NO: 2. An amino acid sequence consisting of DLL4-Fc to which a sequence consisting of 3 of HA tags and 10 of His (3xHA+10His sequence) was added on the N-terminus side and a signal sequence for translocation to the endoplasmic reticulum (ABC sequence) was further added on the N-terminus side was designed, and a nucleotide sequence encoding it was synthesized and used as the DLL4-Fc gene. The amino acid sequence of the 3xHA+10His sequence is shown as SEQ ID NO: 3, and the nucleotide sequence encoding it is shown as SEQ ID NO: 4. The amino acid sequence of the ABC sequence is shown as SEQ ID NO: 5, and the nucleotide sequence encoding it is shown as SEQ ID NO: 6.

The amino acid sequences of human laminin α5-chain E8, β1 -chain E8, and γ1-chain E8 are shown as SEQ ID NOS: 7 to 9, and the nucleotide sequences encoding them are shown as SEQ ID NOS: 10 to 12, respectively. An amino acid sequence consisting of each fragment to which the 3xHA+10His sequence was added on the N-terminus side and the ABC sequence was further added on the N-terminus side was designed, and a nucleotide sequence encoding it was synthesized and used as a gene for each fragment.

The amino acid sequence of GFP is shown as SEQ ID NO: 13, and the nucleotide sequence encoding it is shown as SEQ ID NO: 14. The nucleotide sequence of SEQ ID NO: 14 was synthesized and used as the GFP gene.

Eight types of human-derived proteins classified into the protein disulfide isomerase (PDI) family (ERdj5, ERp27, ERp44, AGR2, AGR3, TNXDC12, ERp28, and TMX1, hereinafter each referred to simply as "PDI" for convenience of explanation) were selected for co-expression. It is considered that no homologues are present in plants for all of these 8 types of PDIs. The amino acid sequences of these 8 types of PDIs and the nucleotide sequences encoding them can be obtained from public databases such as NCBI or from technical literature such as patent documents. The nucleotide sequences of the human ERp27 and TMX1 genes and the amino acid sequences of ERp27 and TMX1 encoded by the genes are as described above. An amino acid sequence consisting of each PDI to which a sequence consisting of a YPYD sequence and a FLAG tag (YPYD+FLAG sequence) was added on the N-terminal side thereof and an endoplasmic reticulum translocation signal (ABC sequence) was further added on the N-terminal side thereof was designed, and a nucleotide sequence encoding it was synthesized and used as a gene for each PDI. The amino acid sequence of the YPYD+FLAG sequence is shown as SEQ ID NO: 15, and the nucleotide sequence encoding it is shown as SEQ ID NO: 16.

Each gene was ligated with pRI201-AN, a binary vector for *Agrobacterium* infection, to obtain a binary vector containing each gene. For human laminin 511E8, expression cassettes of the three fragments of the genes were obtained from the binary vectors containing them, and ligated, and the ligation product was inserted into pR1201 - AN between the L-border and R-border to obtain a binary vector containing a set of those genes. With a binary vector containing each gene, *Agrobacterium tumefaciens* EHA1 05 was transformed to obtain the *Agrobacterium* bacterium containing each gene.

### (3) Culture of Agrobacterium bacterium

To 20 ml of the YEP medium (1% yeast extract, 1% peptone, and 0.5% sodium chloride), 50 mg/l of antibiotic, kanamycin, was added, and the *Agrobacterium* bacterium containing each gene was inoculated in the medium, and cultured overnight at 28°C with shaking. The culture medium was centrifuged at 4,000 rcf and 22°C for 10 minutes to collect the bacterial cells. The bacterial cells were suspended in the MES buffer (1.952 g/l MES, 2.033 g/l MgCl₂·6H₂O, pH 5.7), and the density was adjusted to an OD600 of 0.3 to obtain a bacterial cell suspension for infiltration treatment. When the target protein gene and PDI gene are co-expressed, the cell suspension of *Agrobacterium* bacterium containing each gene was mixed so that the OD600 of mixture should be 0.3 after mixing to obtain a mixed bacterial cell suspension for the infiltration treatment.

### (4) Infiltration treatment and sampling

The bacterial cell suspension or mixed bacterial cell suspension for infiltration treatment was filled into a 1-mL syringe and extruded to infiltrate into the tobacco leaf. This operation was repeated until the *Agrobacterium* suspension penetrated into the epidermis of the entire leaf as the infiltration treatment (infection treatment). The infiltration treatment was performed for about two to three leaves for each target protein. The tobacco plants subjected to the infiltration treatment were cultivated in an artificially lighted hydroponic system (20°C, daylength of 16 hours, 1,000 ppm CO₂). Approximately 10 g of leaf tissue was sampled 5 to 7 days after the infection treatment and stored at -20°C.

### (5) Extraction and purification

The tobacco leaves (10 g) obtained above were disrupted by using a mortar chilled at -80°C for 1 hour, and 6 times the weight of the leaves of an extraction buffer (50 mM Na phosphate, 500 mM NaCl, 200 mM sodium L-ascorbate (V.C.), pH 8.5) was added for extraction. To adjust the pH of the extract to pH 8.5, 1.0 M NaOH was added to the extraction mixture containing the disrupted leaves. The mixture was centrifuged at 4°C and 10,000 rcf for 30 minutes. The supernatant was collected by decantation and filtered through a 0.22 µm filter. The filtrate was subjected to protein G purification (Table 1, for DLL4-Fc) or His-affinity purification (Table 2, for laminin 511E8 and GFP), and the eluate was collected.

**[Table 1]**

| Table 1 Protein G purification | |
|---|---|
| System: | |
| Resin: | Protein G Sepharose^{™} 4 Fast Flow(GE Healthcare Life Sciences, 17-0618-01) |
| | |
| Volume : | 50 *µ* L |
| Temp : | 4°C |
| Load sample: | Tobacco extract |
| Load volume: | 40 mL |
| Binding buffer (A): | 50 mM Na phosphate, 500 mM NaCl, pH 8.5 |
| Elution buffer (B): | Gly-HCl /pH2.8 + 1 M Tris |
| Re-equilibrium: | 100%A, 10 CV |
| Elution: | 100%B / 2 CV |

**[Table 2]**

| Table 2 His-affinity purification | |
|---|---|
| System: | |
| Resin: | HisTALON^{™} Metal Affinity Resin (TAKARA BIO INC., #635502) |
| Volume : | 0.5 mL |
| Temp : | 4°C |
| Load sample: | Tobacco extract |
| Load volume: | 40 mL |
| Binding buffer (A): | 50 mM Na phosphate, 500 mM NaCl, pH 8.5 |
| Elution buffer (B): | 50 mM Na phosphate, 500 mM NaCl, 250 mM imidazole, pH 8.5 |
| Re-equilibrium: | 100%A, 10 CV |
| Elution: | 100%B / 5 CV |

### 2. Results

### (1) Co-expression of DLL4-Fc or Laminin51 1 E8 and PDI

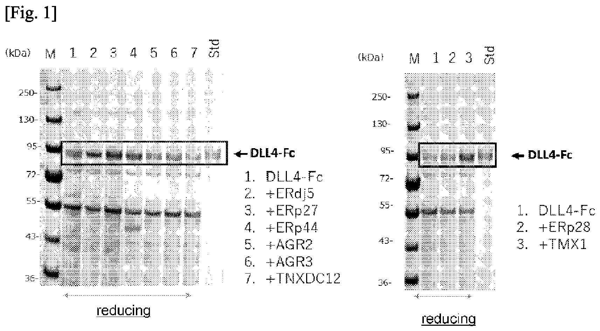

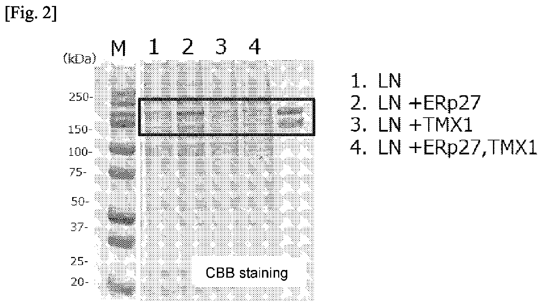

The eluate for DLL4-Fc was subjected to reducing SDS-PAGE, and DLL4-Fc was detected by Western blotting using anti-Fc antibody. The eluate for laminin 511E8 was subjected to non-reducing SDS-PAGE, and laminin 511E8 was detected by CBB staining. The results are shown in Figs. 1 and 2. When DLL4-Fc and each PDI were co-expressed, an increase in DLL4-Fc expression amount was confirmed when the co-expression was performed with ERp27 or TMX1 (Fig. 1). Similarly, when laminin 511E8 and ERp27 or TMX1 were co-expressed, a marked increase in laminin 511E8 expression amount was observed in the co-expression with ERp27, and a slight increase in laminin 511E8 expression amount was observed in the co-expression with TMX1 (Fig. 2). These results indicate that expression of ERp27 or TMX1 is effective for improving expression of target proteins (e.g., secretory proteins that are secreted through the endoplasmic reticulum) in plants.

### (2) Co-expression of GFP and PDI

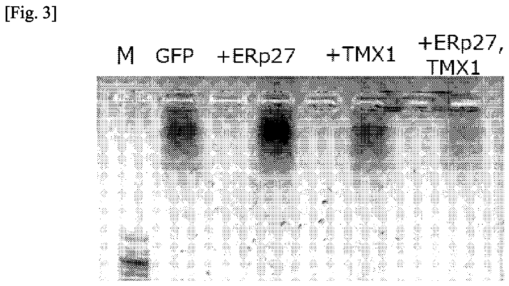

The extract for GFP was electrophoresed on agarose gel and colored with blue light to detect GFP. The results are shown in Fig. 3. Increase in GFP expression amount was confirmed for co-expression with ERp27 (Fig. 3). This result revealed that expression of ERp27 is effective for improving expression of target proteins in plants through or not through the ER.

### Example 2: Expression of protein using tobacco plant expressing ERp27 and POFUT1 as host

### 1. Materials and methods

### (1) Cultivation of tobacco plant and preparation for agroinfiltration treatment

Tobacco plants with about 12 developed true leaves were obtained in the same manner as described in Example 1, (1). The obtained tobacco plants were used for the following infiltration treatment.

### (2) Construction of vector and introduction into Agrobacterium bacterium

DLL4-Fc was selected as the target protein. DLL4-Fc was expressed with a signal for translocation to endoplasmic reticulum as a secretory protein secreted through the endoplasmic reticulum (ER). The amino acid sequence of DLL4-Fc is shown as SEQ ID NO: 1, and the nucleotide sequence encoding it is shown as SEQ ID NO: 2. An amino acid sequence consisting of DLL4-Fc to which a signal for translocation to endoplasmic reticulum (ABC sequence) was added on the N-terminus side (SEQ ID NO: 26) was designed, and a nucleotide sequence encoding it was synthesized and used as the DLL4-Fc gene. The amino acid sequence of the ABC sequence is shown as SEQ ID NO: 5, and the nucleotide sequence encoding it is shown as SEQ ID NO: 6.

Human ERp27 was selected for co-expression. The nucleotide sequence of the human ERp27 gene and the amino acid sequence of ERp27 encoded by the gene are as described above. An amino acid sequence consisting of ERp27 to which a sequence consisting or a YPYD sequence and a FLAG tag (YPYD+FLAG sequence) was added on the N-terminal side thereof, and a signal for translocation to endoplasmic reticulum (ABC sequence) was further added on the N-terminal side thereof was designed, and a nucleotide sequence encoding it was synthesized and used as the ERp27 gene. The amino acid sequence of the YPYD+FLAG sequence is shown as SEQ ID NO: 15, and the nucleotide sequence encoding it is shown as SEQ ID NO: 16.

Human POFUT1 was also selected for co-expression. The nucleotide sequence of the human POFUT1 gene and the amino acid sequence of POFUT1 encoded by the gene are as described above. An amino acid sequence consisting of POFUT1 (mature protein) to which a sequence consisting of a YPYD sequence and a FLAG tag (YPYD+FLAG sequence) was added on the N-terminal side thereof, and an endoplasmic reticulum translocation signal (ABC sequence) was further added on the N-terminal side thereof was designed (SEQ ID NO: 27), and a nucleotide sequence encoding it was synthesized and used as the POFUT1 gene. The amino acid sequence of the YPYD+FLAG sequence is shown as SEQ ID NO: 15, and the nucleotide sequence encoding it is shown as SEQ ID NO: 16.

The DLL4-Fc gene, ERp27 gene, and POFUT1 gene were each introduced into *Agrobacterium tumefaciens* EHA105 in the same manner as described in Example 1, 1, (2) to obtain *Agrobacterium* bacterium containing each gene.

### (3) Culture of Agrobacterium bacterium

The *Agrobacterium* bacterium containing each gene was cultured in the same manner as in Example 1, 1, (3), and mixed bacterial cell suspensions for infiltration treatment (i.e., mixed bacterial cell suspension of *Agrobacterium* bacteria having the DLL4-Fc gene and ERp27 gene, respectively, and mixed bacterial cell suspension of *Agrobacterium* bacteria having the DLL4-Fc gene, ERp27 gene and POFUT1 gene, respectively) were obtained.

### (4) Infiltration treatment and sampling

In the same manner as in Example 1, 1, (4), the infiltration treatment (infection treatment) was performed with the mixed bacterial cell suspensions for the infiltration treatment, and leaf tissue was sampled 6 days after the infection treatment and stored.

### (5) Extraction and purification

In the same manner as in Example 1, 1, (5), DLL4-Fc was extracted from the tobacco leaves and subjected to protein G purification, and the eluate was collected.

### 2. Results

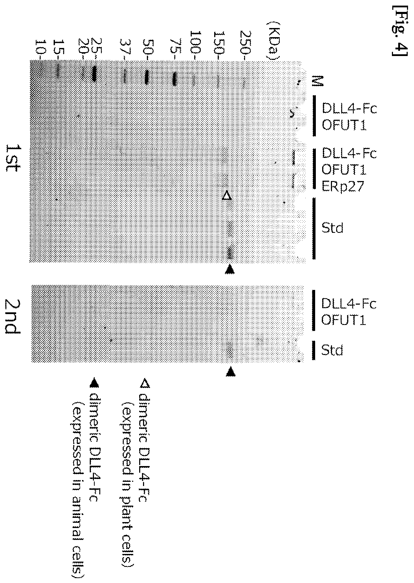

The eluate for DLL4-Fc was subjected to non-reducing SDS-PAGE using a stain-free gel, trihalo compounds were bound to the proteins by UV irradiation, and then the proteins were visualized as fluorescence images under UV irradiation. As a standard (Std) of DLL4-Fc, a dimeric DLL4-Fc expressed in animal cells was used. The results are shown in Fig. 4. When DLL4-Fc was co-expressed with POFUT1 alone, no bands of dimeric DLL4-Fc could be confirmed, whereas when DLL4-Fc was co-expressed with ERp27 and POFUT1, a band of dimeric DLL4-Fc was confirmed (Fig. 4). These results again demonstrated that expression of ERp27 is effective for improving expression of a target protein in plants. It is also considered that expression of POFUT1 improved the folding of DLL4-Fc, and thereby DLL4-Fc was efficiently produced.

### Industrial applicability

According to the present invention, production of proteins using plants as an expression host can be improved.

### <Description of the Sequence Listing>

SEQ ID NO: 1, amino acid sequence of DLL4-Fc
SEQ ID NO: 2, nucleotide sequence encoding DLL4-Fc
SEQ ID NO: 3, amino acid sequence of an additional sequence consisting of 3 HA tags and 10 His
SEQ ID NO: 4, nucleotide sequence encoding the additional sequence consisting of 3 HA tags and 10 His
SEQ ID NO: 5, amino acid sequence of a signal sequence for translocation to the endoplasmic reticulum
SEQ ID NO: 6, nucleotide sequence encoding the signal sequence for translocation to the endoplasmic reticulum
SEQ ID NO: 7, amino acid sequence of human laminin α5E8
SEQ ID NO: 8, amino acid sequence of human laminin β1E8
SEQ ID NO: 9, amino acid sequence of human laminin γ1E8
SEQ ID NO: 10, nucleotide sequence encoding human laminin α5E8
SEQ ID NO: 11, nucleotide sequence encoding human laminin β1E8
SEQ ID NO: 12, nucleotide sequence encoding human laminin γ1E8
SEQ ID NO: 13, amino acid sequence of GFP
SEQ ID NO: 14, nucleotide sequence encoding GFP
SEQ ID NO: 15, amino acid sequence of an additional sequence consisting of the YPYD sequence and FLAG tag
SEQ ID NO: 16, nucleotide sequence encoding the additional sequence consisting of the YPYD sequence and FLAG tag
SEQ ID NO: 17, amino acid sequence of HA tag
SEQ ID NO: 18, amino acid sequence of HAΔYA tag
SEQ ID NO: 19, amino acid sequence of HAΔVPDYA tag
SEQ ID NO: 20, nucleotide sequence of human ERp27 gene
SEQ ID NO: 21, amino acid sequence of human ERp27
SEQ ID NO: 22, nucleotide sequence of human TMX1 gene
SEQ ID NO: 23, amino acid sequence of human TMX1
SEQ ID NO: 24, nucleotide sequence of human POFUT1 gene (part encoding mature protein)
SEQ ID NO: 25, amino acid sequence of human POFUT1 (mature protein)
SEQ ID NO: 26, amino acid sequence of DLL4-Fc having an additional sequence
SEQ ID NO: 27, amino acid sequence of human POFUT1 having an additional sequence

## Claims

1. A method for producing a target protein, wherein
the method comprises a step of expressing a gene encoding the target protein in a plant having the gene,
wherein the plant has been modified so as to express ERp27 and/or TMX1, with the proviso that the target protein is expressed in a form containing a signal peptide at the N-terminus when the plant has been modified to express only TMX1 among ERp27 and TMX1.

2. The method according to claim 1, wherein the target protein is expressed in a form containing a signal peptide at the N-terminus.

3. The method according to claim 1 or 2, wherein the signal peptide has such a function that the target protein expressed in a form having the signal peptide at the N-terminus in the plant is translocated or localized to the endoplasmic reticulum.

4. The method according to any one of claims 1 to 3, wherein the ERp27 is a protein described in the following (a), (b), or (c):
(a) a protein comprising the amino acid sequence shown as SEQ ID NO: 21;
(b) a protein comprising an amino acid sequence of SEQ ID NO: 21 but which includes substitution, deletion, insertion, and/or addition of 1 to 10 amino acid residues, and having a function of increasing production of the target protein by the plant when expressed in the plant; or
(c) a protein comprising an amino acid sequence having at least 90% identity to the amino acid sequence shown as SEQ ID NO: 21, and having a function of increasing production of the target protein by the plant when expressed in the plant.

5. The method according to any one of claims 1 to 4, wherein the TMX1 is a protein described in the following (a), (b), or (c):
(a) a protein comprising the amino acid sequence shown as SEQ ID NO: 23;
(b) a protein comprising an amino acid sequence of SEQ ID NO: 23 but which includes substitution, deletion, insertion, and/or addition of 1 to 10 amino acid residues, and having a function of increasing production of the target protein by the plant when expressed in the plant; or
(c) a protein comprising an amino acid sequence having at least 90% identity to the amino acid sequence shown as SEQ ID NO: 23, and having a function of increasing production of the target protein by the plant when expressed in the plant.

6. The method according to any one of claims 1 to 5, wherein the plant is a plant of the family *Solanaceae.*

7. The method according to any one of claims 1 to 6, wherein the plant is benthamiana tobacco (*Nicotiana benthamiana*) or tobacco (*Nicotiana tabacum*)*.*

8. The method according to any one of claims 1 to 7, which further comprises a step of collecting the target protein.

9. The method according to claim 8, wherein the step of collecting comprises a step of extracting the target protein from the plant.

10. The method according to claim 9, wherein the target protein is extracted from a leaf of the plant.

11. The method according to claim 9 or 10, wherein
the extracting is performed in the presence of an active ingredient, and
the active ingredient is an antioxidant and/or a metal sequestering agent.

12. The method according to claim 11, wherein the active ingredient is L-ascorbic acid.

13. The method according to any one of claims 9 to 12, wherein the target protein is expressed and extracted in a form containing an HA tag.

14. The method according to claim 13, wherein the HA tag is a peptide described in the following (a), (b), or (c):
(a) a peptide comprising the amino acid sequence shown as SEQ ID NO: 17, 18, or 19;
(b) a peptide comprising an amino acid sequence of SEQ ID NO: 17, 18, or 19 but which includes substitution, deletion, insertion, and/or addition of 1 to 5 amino acid residues, containing a tyrosine residue, and having a function of increasing production of the target protein by the plant when added to the target protein; or
(c) a peptide comprising an amino acid sequence having at least 50% identity to the amino acid sequence shown as SEQ ID NO: 17, 18, or 19, containing a tyrosine residue, and having a function of increasing production of the target protein by the plant when added to the target protein.

15. The method according to claim 13 or 14, wherein the HA tag contains 1 to 5 tyrosine residues.

16. The method according to any one of claims 1 to 15, wherein the target protein is a heterologous protein.

17. The method according to any one of claims 1 to 16, wherein the target protein is a human-derived protein.

18. The method according to any one of claims 1 to 17, wherein the target protein is a multimeric protein.

19. The method according to any one of claims 1 to 18, wherein the target protein is one or more types of proteins selected from an extracellular protein, an antibody-related molecule, a Notch ligand, and GFP.

20. The method according to any one of claims 1 to 19, wherein the target protein is laminin or a partial sequence thereof.

21. The method according to any one of claims 1 to 20, wherein the target protein is laminin 511E8.

22. The method according to any one of claims 1 to 19, wherein the target protein is an Fc fusion protein.

23. The method according to any one of claims 1 to 19 and 22, wherein the target protein is DLL4-Fc.

24. The method according to any one of claims 9 to 23, wherein the step of collecting further comprises a step of collecting the target protein from an extract obtained by the extracting.

25. The method according to any one of claims 1 to 24, wherein the plant has been further modified to express a chaperone.

26. The method according to claim 25, wherein the chaperone is O-fucosyltransferase 1.

27. The method according to claim 25 or 26, wherein the chaperone is a protein described in the following (a), (b), or (c):
(a) a protein comprising the amino acid sequence shown as SEQ ID NO: 25;
(b) a protein comprising an amino acid sequence of SEQ ID NO: 25 but which includes substitution, deletion, insertion, and/or addition of 1 to 10 amino acid residues, and having an O-fucosyltransferase 1 activity; or
(c) a protein comprising an amino acid sequence having at least 90%identity to the amino acid sequence shown as SEQ ID NO: 25 and having an O-fucosyltransferase 1 activity.
